# EUROPEAN PATENT APPLICATION

(11) **EP 4 424 662 A1**
(43) Date of publication of application: **04.09.2024**
(21) Application number: 22885815.5
(22) Date of filing: 21.10.2022
(51) Int. Cl.: C07C 51/087, C07C 55/10, C07D 307/60, B01J 8/04

(54) **MALEIC ANHYDRIDE HYDROGENATION METHOD AND SUCCINIC ACID PRODUCTION METHOD COMPRISING SAME**

(30) Priority: 27.10.2021 CN 202111253762; 29.10.2021 CN 202111272561
(71) Applicant: CHINA PETROLEUM & CHEMICAL CORPORATION, Chaoyang District Beijing 100728 (CN); BEIJING RESEARCH INSTITUTE OF CHEMICAL INDUSTRY, CHINA PETROLEUM & CHEMICAL CORPORATION, Chaoyang District Beijing 100013 (CN)
(72) Inventor: WU, Changjiang, Beijing 100013 (CN); WANG, Guoqing, Beijing 100013 (CN); LI, Dongfeng, Beijing 100013 (CN); GUO, Liang, Beijing 100013 (CN); ZHANG, Lijun, Beijing 100013 (CN); LI, Yan, Beijing 100013 (CN); PENG, Hui, Beijing 100013 (CN); LUO, Shujuan, Beijing 100013 (CN); TIAN, Jun, Beijing 100013 (CN); SHI, Huimin, Beijing 100013 (CN); ZHU, Yuehui, Beijing 100013 (CN); ZHANG, Dongshun, Beijing 100013 (CN); YE, Jieming, Beijing 100013 (CN); LI, Chunfang, Beijing 100013 (CN); LU, Shuliang, Beijing 100013 (CN); XU, Yang, Beijing 100013 (CN); SHI, Qian, Beijing 100013 (CN)
(74) Representative: Hoffmann Eitle
(86) International application number: PCT/CN2022/126710
(87) International publication number: WO 2023/071938

(57) **Abstract**

The present invention relates to a process for producing succinic anhydride by maleic anhydride hydrogenation, comprising (1) feeding a maleic anhydride solution and a hydrogen raw material respectively to a first-stage hydrogenation reactor from an upper liquid-phase feed port and a top gas-phase feed port of the first-stage hydrogenation reactor for a first-stage hydrogenation reaction to obtain a first-stage hydrogenation product; (2) feeding the first-stage hydrogenation product to a second-stage hydrogenation reactor for a second-stage hydrogenation reaction to obtain a second-stage hydrogenation product, optionally, before entering the second-stage hydrogenation reactor, subjecting the first-stage hydrogenation product to a first-stage gas-liquid separation to obtain a first-stage gas phase and a first-stage liquid phase, and then feeding the first-stage gas phase and the first-stage liquid phase respectively from a top gas-phase feed port and an upper liquid-phase feed port of the second-stage hydrogenation reactor; and (3) subjecting the second-stage hydrogenation product to a second-stage gas-liquid separation to obtain a second-stage gas phase and a second-stage liquid phase, returning a part of the second-stage liquid phase to step (1) to be mixed with the maleic anhydride solution for the first-stage hydrogenation reaction, and optionally, using part or all of the second-stage gas phase as a circulating hydrogen. The present invention also relates to a process for producing succinic acid comprising such process, a liquid-phase hydrogenation reaction system, and a system for producing succinic acid comprising such system.

## Description

### Technical field

The present invention relates to a process for producing succinic anhydride by maleic anhydride hydrogenation. The present invention also relates to a process for producing succinic acid comprising such process. The present invention further relates to a liquid phase hydrogenation reaction system and a system for producing succinic acid comprising such system.

### Background art

Succinic acid (C₄H₆O₄) is a colorless or white, odorless solid with a sour taste. It can react with a base, can also undergo reactions such as esterification and reduction, can be dehydrated by heating to form succinic anhydride, and can undergo nucleophilic substitution reaction wherein hydroxyls are substituted by halogen atoms, amine compounds, acyl groups, etc. It is an important fine chemical product and organic synthesis intermediate. Succinic acid and its derivatives are useful platform chemicals, and are widely used in the manufacture of polymers, fuel additives, inks and cosmetics, and as additives in foods and pharmaceuticals. Succinic acid is an important monomer in the production of polybutylene succinate (PBS). With the rise of degradable plastic PBS resin worldwide, the production of succinic acid has received more and more widespread attention, with a huge market potential and a broad development prospect.

The process for producing succinic acid mainly includes microbial fermentation, electrochemical synthesis and maleic anhydride catalytic hydrogenation. The process of preparing succinic acid by microbial fermentation is cumbersome in process, discharges a large amount of wastewater, and has high cost of production and separation. The process of preparing succinic acid by electrochemical synthesis does not achieve a high conversion rate and consumes a lot of power, it is difficult to maintain the electrolytic tank, the electrodes are seriously corroded, and sewage is discharged in a large amount, thus the process is not suitable for large-scale production. The catalytic hydrogenation process is to hydrogenate maleic anhydride or maleic acid under the action of a catalyst to generate succinic anhydride, which is then hydrolyzed to obtain succinic acid. This process to produce succinic acid has the advantages of high conversion rate, high product purity, no obvious side reactions, environmental friendliness, and the like. It is currently the most widely used succinic acid synthesis process in industry.

Succinic anhydride is an important organic synthesis intermediate and fine chemical raw material. It can undergo hydrolysis, alcoholysis, esterification, halogenation, acylation and other reactions, and is widely used in fields such as medicine, pesticides, food, petrochemicals, building materials, synthetic resins, dyes and the like. At present, there are few manufacturers producing succinic anhydride worldwide, and the output is not large, while succinic anhydride is widely used, resulting in a shortage of succinic anhydride worldwide. Enterprises producing succinic anhydride in China are small in scale and have low output, which is far from meeting the domestic demand for succinic anhydride. In particular, high-purity succinic anhydride is almost entirely imported.

According to the source of raw materials, the main processs for preparing succinic anhydride include maleic anhydride hydrogenation process, succinic acid dehydration process, acetylene carbonylation process, etc. The current industrial production processs are mainly maleic anhydride hydrogenation process and succinic acid dehydration process.

The succinic acid dehydration process requires obtaining succinic acid raw material and then dehydrating it under certain conditions, and it is the earliest process for producing succinic anhydride. Since there are few sources of succinic acid, which mainly include production by catalytic hydrogenation or electrolysis of maleic anhydride, the production cost is high. As is seen from the synthesis route, dehydration of succinic acid to prepare succinic anhydride is the reverse reaction of hydrolysis of succinic anhydride, while succinic anhydride is easily hydrolyzed to produce succinic acid, thus this process has no development prospect either in terms of rationality of the process route or in terms of economy.

At present, most of the production of the succinic anhydride in the world adopts the maleic anhydride hydrogenation process. Maleic anhydride is directly hydrogenated in an organic solvent to produce succinic anhydride. The process has a high conversion rate and yield, no obvious side reactions, and a good product purity. However, since the hydrogenation of maleic anhydride to produce succinic anhydride is a strongly exothermic reaction (ΔH = -128kJ/mol), the adiabatic temperature rise of the reaction is large, which can easily cause organic matter to polymerize and coke on the catalyst surface, thereby reducing the catalyst activity. At the same time, due to the accumulation of reaction heat, it is easy to cause the temperature of the catalyst bed to rise sharply, thereby temperature runaway phenomenon occurs. Therefore, how to take effective measures to reduce the heat released by the reaction is the focus and difficulty of the maleic anhydride hydrogenation process.

CN103570650A discloses a process for continuously producing succinic anhydride and co-producing succinic acid through maleic anhydride hydrogenation. Two stages of hydrogenation reactors are used. At the outlet of the primary hydrogenation reactor, after material heat exchange, part of the reaction liquid enters the secondary hydrogenation reactor, and the remaining reaction liquid is mixed with the solution of the raw material maleic anhydride and then re-enters the primary hydrogenation reactor device. Although this process can achieve a certain reaction heat removal effect, since the material at the outlet of the primary reactor still comprises a certain amount of maleic anhydride, when this material is recycled to the inlet of the primary reactor, the amount of maleic anhydride entering the primary reactor does not decrease significantly, and the amount of heat released by the reaction in the primary reactor does not decrease significantly, thereby there is a great pressure in terms of removal of the reaction heat of the reactor.

CN107253938A discloses a production process for preparing high-purity succinic anhydride through direct hydrogenation of maleic anhydride. The direct hydrogenation of maleic anhydride adopts a fixed bed for hydrogenation, hydrogen is recycled and only a small amount of hydrogen is added as a supplement, the molar ratio of hydrogen to maleic anhydride is 800-1000, hydrogen is added through four stages of cold hydrogen, so that maleic anhydride can fully react during the process of falling from the top, reducing the formation of other heavy components such as butyrolactone, then maleic anhydride can fully participate in the reaction, and the main components in the reaction product are succinic anhydride, maleic anhydride and butyrolactone. This process can achieve a certain reaction heat removal effect, but due to the relatively large amount of circulating hydrogen, the energy consumed by the compressor for circulating hydrogen is relatively large, and meanwhile, the amount of hydrogen entering the reactor is large, correspondingly the reactor volume needs to be increased, resulting in increased investment and increased energy consumption.

Therefore, there is an urgent need to develop a maleic anhydride hydrogenation reaction process to effectively remove the heat released by the reaction and at the same time solve the current problems of too large hydrogen/anhydride ratio, large investment, and high energy consumption.

The processes for the production of maleic anhydride mainly include benzene oxidation process and n-butane oxidation process according to the raw material route. With the development of large-scale intensification of maleic anhydride production, the post-treatment of maleic anhydride is increasingly inclined to adopt solvent absorption and desorption technology. Typical solvent absorption and desorption processes include the Huntsman process, the Conser process and the ALMA process. The first two use dibutyl phthalate (DBP) as a solvent, and the last one uses diisobutyl hexahydrophthalate (DIBP) as a solvent. Regardless of the process, the crude maleic anhydride after absorption and desorption needs to be distilled and purified multiple times before the refined maleic anhydride product can be obtained, and multiple separation towers are operated under reduced pressure, thereby resulting in large equipment investment, complex processes, and long process.

Moreover, the absorption solvent used to produce maleic anhydride and the solvent for maleic anhydride hydrogenation reaction are different solvents, so that the existing process of producing maleic anhydride cannot be matched with the maleic anhydride hydrogenation reaction process, and they are two independent processes. In the process of producing maleic anhydride and in the process of producing succinic anhydride, the use of different solvents results in two times of solvent desorption, purification and recycle, as well as two times of product separation and refinement. Two independent sets of solvent separation and recovery and product separation and refinement systems are required. Therefore, the existing process route has problems such as long process, large investment, high energy consumption, large loss of raw materials and auxiliary materials, high operating costs, etc. For companies with C4 resources or benzene resources, there is an urgent need to optimize and improve the process for the production of succinic acid from butane or benzene as a raw material.

Therefore, there is still a need for a process and system for producing succinic anhydride by hydrogenation of maleic anhydride that can effectively remove the heat of reaction and reduce the molar ratio of hydrogen to maleic anhydride, as well as a process and system for producing succinic acid by combining the process and system for producing succinic anhydride by hydrogenation of maleic anhydride. In addition, there is still a need for a process and system for producing succinic acid with simplified and improved separation operations of maleic anhydride, which can also effectively integrate the process of producing maleic anhydride and the process of hydrogenating maleic anhydride to produce succinic anhydride to achieve continuous production of succinic acid.

### Summary of the invention

In order to solve many problems in the prior art, for example, in hydrogenation reaction processes and systems such as hydrogenation of maleic anhydride to succinic anhydride, large exothermicity, difficult heat removal, high molar ratio of hydrogen to maleic anhydride, large investment in production process, high energy consumption, etc., an object of the present invention is to provide a process and system for producing succinic anhydride by hydrogenation of maleic anhydride, which process and system have the characteristics of easy removal of reaction heat, low molar ratio of hydrogen to maleic anhydride, low investment and low energy consumption, and can achieve the desired maleic anhydride conversion rate and succinic anhydride selectivity.

Another object of the present invention is to provide a process and system for producing succinic acid comprising the process and system for producing succinic anhydride by hydrogenation of maleic anhydride, which can achieve the above effects such as easy removal of reaction heat and low molar ratio of hydrogen to maleic anhydride, and obtain succinic acid having the desired quality.

A further object of the present invention is to provide a process and system for the whole-process production of succinic acid from butane/benzene as raw materials. This process and system simplify and improve the maleic anhydride separation operation process, can realize that the absorption solvent used in the production of maleic anhydride and the solvent for the maleic anhydride hydrogenation reaction are the same and applicable to each other, and obtain succinic acid having the desired quality, and have the characteristics of a simple process, saving investment, strong applicability, easy control and the like. The process and system realize the combined use of the maleic anhydride separation process and the maleic anhydride hydrogenation process.

The objects of the present invention are achieved through one or more of the following aspects.

In a first aspect, the present invention provides a process for producing succinic anhydride by hydrogenation of maleic anhydride, comprising the following steps:
(1) feeding a maleic anhydride solution and a hydrogen raw material to a first-stage hydrogenation reactor from an upper liquid-phase feed port and a top gas-phase feed port of the first-stage hydrogenation reactor respectively for a first-stage hydrogenation reaction to obtain a first-stage hydrogenation product;
(2) feeding the first-stage hydrogenation product to a second-stage hydrogenation reactor for a second-stage hydrogenation reaction to obtain a second-stage hydrogenation product; optionally, before entering the second-stage hydrogenation reactor, subjecting the first-stage hydrogenation product to a first-stage gas-liquid separation to obtain a first-stage gas phase and a first-stage liquid phase, and then feeding the first-stage gas phase and the first-stage liquid phase from a top gas-phase feed port and an upper liquid-phase feed port of the second-stage hydrogenation reactor respectively; and
(3) subjecting the second-stage hydrogenation product to a second-stage gas-liquid separation to obtain a second-stage gas phase and a second-stage liquid phase, and returning a part of the second-stage liquid phase to step (1) to be mixed with the maleic anhydride solution for the first-stage hydrogenation reaction, optionally using part or all of the second-stage gas phase as a circulating hydrogen.

In a second aspect, the present invention provides a process for producing succinic acid comprising the process for the hydrogenation of maleic anhydride according to the present invention.

In a third aspect, the present invention provides a liquid phase hydrogenation reaction system, which system comprises:
a first-stage hydrogenation reactor, comprising a top gas-phase feed port, an upper liquid-phase feed port and a bottom discharge port;
a first-stage hydrogenation product cooler and a first-stage gas-liquid separator, which are successively connected in series to the bottom discharge port of the first-stage hydrogenation reactor;
a second-stage hydrogenation reactor, which is connected in series with the first-stage gas-liquid separator, and comprises a top gas-phase feed port, an upper liquid-phase feed port, and a bottom discharge port;
a second-stage gas-liquid separator, which is connected in series with the bottom discharge port of the second-stage hydrogenation reactor; and
a liquid-phase raw material supply pipeline, which is connected to both the upper liquid-phase feed port of the first-stage hydrogenation reactor and the upper liquid-phase feed port of the second-stage hydrogenation reactor.

In a fourth aspect, the present invention provides a system for producing succinic acid from butane and/or benzene comprising a liquid phase hydrogenation reaction system according to the present invention as a maleic anhydride hydrogenation reaction unit.

In a fifth aspect, the present invention provides the use of the liquid phase hydrogenation reaction system according to the present invention in a succinic acid production system.

The technical solution of the present invention has at least the following benefits:
by adopting the process of the present invention, the heat removal manner for maleic anhydride hydrogenation is improved, the heat generated by maleic anhydride hydrogenation can be effectively removed, the hydrogen/anhydride ratio of the system is reduced, investment is reduced, and desired maleic anhydride conversion rate and succinic anhydride selectivity are obtained; the concentration of maleic anhydride in the maleic anhydride solution does not need to be too low, which reduces the amount of solvent used so that the energy consumption of subsequent solvent recovery is reduced; in the present invention, after the first-stage hydrogenation reaction, following cooling and gas-liquid separation, all the gas phase is fed to the second-stage reactor, which is conducive to effectively removing the reaction heat generated by the second-stage hydrogenation reaction; the operation conditions for the reaction of the present invention are mild, the reactor can be operated at 40°C, which greatly reduces the severity of the reaction, and the temperature rise of the reaction bed is low, which is conducive to improving catalyst selectivity and extending catalyst life;
by dividing the maleic anhydride solution into several streams, such as two or three streams, to mix with different materials respectively and then feeding the mixtures into various, such as two or three, hydrogenation reactors, under the same treatment amount, the amount of maleic anhydride entering the first-stage reactor decreases, thereby the heat released by the reaction can be effectively controlled, and the operation is flexible and easy to control;
the maleic anhydride separation unit of the present invention is simpler than the traditional processes and saves equipment investment; the solvent used in the maleic anhydride separation unit is the same as the solvent used for maleic anhydride hydrogenation, and can be a by-product produced by this process itself, and the solvent is recycled and does not need to be purchased, thereby achieving low investment, independent process, and strong practicability; the maleic anhydride separation unit can be operated without vacuum, which not only saves equipment investment, but also helps to save energy consumption; the maleic anhydride separation unit can be satisfied by adjusting the operating conditions such as the absorbent at any time according to the solution concentration or solvent required by the subsequent maleic anhydride hydrogenation reaction, achieving the whole-process controllable operation in the industry; the maleic anhydride separation system effectively removes substances that are easy to cause side reactions, which is beneficial to the production of succinic anhydride;
the succinic anhydride separation unit of the present invention is easy to operate and easy to control;
the succinic acid production process of the present invention has the characteristics of simple process, saving investment, strong applicability, easy control, etc., realizes the combined use of the maleic anhydride separation process, the maleic anhydride hydrogenation process and the succinic anhydride separation process, and obtains the desired overall yield and quality of succinic acid.

### Description of drawings

Figure 1 is a schematic diagram of a maleic anhydride hydrogenation process according to an embodiment of the present invention.
Figure 2 is a schematic diagram of a maleic anhydride hydrogenation process according to another embodiment of the present invention.
Figure 3 is a schematic diagram of a maleic anhydride hydrogenation process according to yet another embodiment of the present invention.
Figure 4 is a schematic diagram of a process for producing succinic acid from butane/benzene according to an embodiment of the present invention.
Figure 5 is a schematic diagram of a process for producing succinic acid from butane/benzene according to another embodiment of the present invention.

### Detailed description of the invention

The endpoints of ranges and any values disclosed herein are not limited to the precise ranges or values, but these ranges or values are to be understood to include values approaching such ranges or values. For numerical ranges, the endpoint values of each range, the endpoint values of each range and individual point values, and the individual point values can be combined with each other to obtain one or more new numerical ranges. These numerical ranges shall be deemed to be specifically disclosed herein.

The present invention will be described in detail below with reference to specific drawings and examples. It is necessary to point out here that the following examples are only used to further illustrate the present invention and cannot be understood as limiting the scope of the present invention. Some non-essential improvements and adjustments made by a person skilled in the art to the present invention according to the contents of the invention still fall within the protection scope of the present invention.

### Maleic anhydride hydrogenation process

The present invention provides a process for producing succinic anhydride by hydrogenation of maleic anhydride, comprising the following steps:
(1) feeding a maleic anhydride solution and a hydrogen raw material to a first-stage hydrogenation reactor from an upper liquid-phase feed port and a top gas-phase feed port of the first-stage hydrogenation reactor respectively for a first-stage hydrogenation reaction to obtain a first-stage hydrogenation product;
(2) feeding the first-stage hydrogenation product to a second-stage hydrogenation reactor for a second-stage hydrogenation reaction to obtain a second-stage hydrogenation product; optionally, before entering the second-stage hydrogenation reactor, subjecting the first-stage hydrogenation product to a first-stage gas-liquid separation to obtain a first-stage gas phase and a first-stage liquid phase, and then feeding the first-stage gas phase and the first-stage liquid phase from a top gas-phase feed port and an upper liquid-phase feed port of the second-stage hydrogenation reactor respectively; and
(3) subjecting the second-stage hydrogenation product to a second-stage gas-liquid separation to obtain a second-stage gas phase and a second-stage liquid phase, and returning a part of the second-stage liquid phase to step (1) to be mixed with the maleic anhydride solution for the first-stage hydrogenation reaction, optionally using part or all of the second-stage gas phase as a circulating hydrogen.

The present invention has no special requirements on the composition of the maleic anhydride solution. According to a preferred embodiment of the present invention, in step (1), the maleic anhydride solution is a mixture of maleic anhydride and a solvent, and the solvent type can be a commonly-used solvent, for example, the solvent is one or more of acetic anhydride, gamma-butyrolactone, dioxane, tetrahydrofuran, aromatic hydrocarbons, ethyl acetate, C4 dibasic acid esters, ethanol, isopropanol, hexane, cyclohexane, propylene oxide, ketones and ethers.

Generally, increasing the concentration of maleic anhydride in the raw material solution can increase the reactor productivity, but it will easily form an extremely high temperature at the hydrogenation sites on the catalyst surface, causing sintering of active components of the catalyst or polymerization and coking of organic matter. By using the process of the present invention, since a part of the second-stage liquid phase (substantially not comprising maleic anhydride) is returned to step (1) and plays the effect of diluting the maleic anhydride solution and reduce heat generation, the maleic anhydride concentration in the incoming maleic anhydride solution does not need to be too low, which reduces the amount of solvent used so that the energy consumption of subsequent solvent recovery is reduced. According to a preferred embodiment of the present invention, the maleic anhydride solution has a maleic anhydride concentration of 1-90% by weight, preferably 5-40% by weight, more preferably 10-40% by weight.

In the prior art, hydrogen is used to remove the reaction heat. However, the large amount of hydrogen brought by the high ratio of hydrogen to maleic anhydride (i.e., the hydrogen/anhydride ratio) will cause deep hydrogenation of succinic anhydride to produce by-products such as γ-butyrolactone, and will greatly increase the energy consumption of the hydrogen compressor. In the present invention, by recycling a part of the second-stage liquid phase to the first-stage hydrogenation reactor, the reaction heat is effectively reduced, thereby achieving a significant reduction in the hydrogen/anhydride ratio. According to a preferred embodiment of the present invention, the molar ratio of the total amount of hydrogen to the total maleic anhydride in the maleic anhydride solution is 5-100, preferably 5-60, more preferably 10-40, such as 15-35 or 20-30. Herein, a low hydrogen/anhydride ratio can be used to indicate efficient removal of reaction heat.

In the present invention, the hydrogen raw material can be fresh hydrogen or circulating hydrogen. According to a preferred embodiment of the present invention, the hydrogen raw material in step (1) is a mixed hydrogen gas of the circulating hydrogen in step (3) and supplementary hydrogen, so that the reaction heat can be effectively removed and the catalytic efficiency can be improved.

According to a preferred embodiment of the present invention, in step (1), the operation conditions for the first-stage hydrogenation reactor have no special requirements and can be conventional operation conditions, for example, including: a temperature of 30-100°C, preferably 40-80°C, for example, 40°C, 41 °C, 42°C, 43°C, 44°C, 45°C, 46°C, 47°C, 48°C, 49°C, 50°C, etc., and so on, with each reaction temperature being suitable for the present invention; and/or a reaction pressure of 0.1-10MPa, preferably 0.5-5MPa; and/or a space velocity of 0.5-8h⁻¹, preferably 0.5-5h⁻¹, such as 1, 2, 3, 4h⁻¹.

According to the present invention, in step (1), after the raw material maleic anhydride solution is mixed with a part of the liquid phase material from the second-stage hydrogenation reaction of step (3), the mixture is fed to the first-stage hydrogenation reactor from the liquid-phase feed port of the first-stage hydrogenation reactor to be contacted with hydrogen for hydrogenation reaction.

In step (1), the maleic anhydride solution can be divided into several streams, such as two or three streams, which can then be mixed with different materials respectively before entering the various, such as two or three, hydrogenation reactors, so that the amount of maleic anhydride entering the reactor decreases, and the heat released in each reactor is reduced. The operation is flexible and easy to control. In one embodiment, the maleic anhydride solution can be divided into at least two streams, wherein the first stream can be mixed with a part of the second-stage liquid phase recycled in step (3) and then enter the first-stage hydrogenation reactor for the first-stage hydrogenation reaction, and the second stream enters the second-stage hydrogenation reactor for the second-stage hydrogenation reaction. For example, the second stream can be mixed with the first-stage hydrogenation product or the first-stage liquid phase (in the case of performing the first-stage gas-liquid separation) and then used for the second-stage hydrogenation reaction.

According to a preferred embodiment of the present invention, the first stream and the second stream are each 5-95% by weight relative to the raw material maleic anhydride solution; more preferably, the first stream is 20-60% by weight, such as 20-50% by weight, and the second stream is 40-80% by weight, such as 50-80% by weight. As a result, the amount of heat released by each reactor can be reduced, which is very beneficial to heat removal and improves the reaction efficiency.

According to a preferred embodiment of the present invention, in step (2), the operation conditions for the second-stage hydrogenation reactor have no special requirements and can be conventional operation conditions, for example, including: a temperature of 30-100°C, preferably 40-80°C, for example, 40°C, 41 °C, 42°C, 43°C, 44°C, 45°C, 46°C, 47°C, 48°C, 49°C, 50°C, etc., and so on, with each reaction temperature being suitable for the present invention; and/or a pressure of 0.1-10MPa, preferably 0.5-5MPa; and/or a space velocity of 0.5-8h⁻¹, preferably 0.5-5h⁻¹, such as 1, 2, 3, 4h⁻¹.

The present invention mainly lies in the process design. There is no limit to the hydrogenation catalyst, such as the catalyst loaded in the first-stage hydrogenation reactor and the second-stage hydrogenation reactor, and any maleic anhydride hydrogenation catalyst can be used, such as the catalysts described in the Chinese patent applications CN114433100(A) and CN114433127 (A).

According to a preferred embodiment of the present invention, when the gas phase and the liquid phase enter the first-stage hydrogenation reactor and the second-stage hydrogenation reactor, they optionally pass through a distribution device and then contact the catalyst.

In the present invention, after the first-stage reactor, a gas-liquid separation can be arranged as needed, the gas phase and the liquid phase enter the hydrogenation reactor respectively, and preferably pass through a distribution device, so that the materials entering the reactor are more fully contacted, the gas-liquid-solid contact is good, the effective utilization rate of the catalyst is high and the investment is low.

According to an embodiment of the present invention, in step (2), the first-stage hydrogenation product is subjected to the first-stage gas-liquid separation to obtain the first-stage gas phase and the first-stage liquid phase, and the first-stage gas phase and the first-stage liquid phase are respectively fed to the second-stage hydrogenation reactor from the top gas-phase feed port and the upper liquid-phase feed port of the second-stage hydrogenation reactor for the second-stage hydrogenation reaction to obtain the second-stage hydrogenation product.

According to a preferred embodiment of the present invention, in step (2), the first-stage hydrogenation product can be cooled before performing the first-stage gas-liquid separation.

According to a preferred embodiment of the present invention, in step (3), said part of the second-stage liquid phase material can be cooled before being mixed with the maleic anhydride solution and converted into a cooled material, so that the reaction heat can be effectively removed and catalytic efficiency can be improved. Preferably, said part of the second-stage liquid phase material is a material cooled to 30-80°C, preferably cooled to 40-60°C.

According to a preferred embodiment of the present invention, in step (3), 20-90% by weight, preferably 30-70% by weight of the second-stage liquid phase material is returned to step (1) for use as a raw material, and the rest of the second-stage liquid phase is sent as a liquid phase product to a subsequent separation system, such as a succinic anhydride separation unit. As a result, heat can be effectively removed and reaction efficiency can be improved.

According to a preferred embodiment of the present invention, 0.5-2% by weight of the second-stage gas phase material of the second-stage hydrogenation reaction is withdrawn as fuel gas, and the rest of the second-stage gas phase is used as a circulating hydrogen. Preferably, 0.5% to 2% of the second-stage gas phase is withdrawn as fuel gas, and the rest of the second-stage gas phase is cooled and recycled to the first-stage hydrogenation reactor, mixed with supplementary fresh hydrogen, and enters the first-stage hydrogenation reactor.

According to a preferred embodiment of the present invention, part or all of the second-stage gas phase obtained by gas-liquid separation of the second-stage hydrogenation product is recycled to the first-stage hydrogenation reactor for use as a circulating hydrogen, and the rest is used as vent gas, and the second-stage liquid phase material obtained by gas-liquid separation of the second-stage hydrogenation product is partially recycled to the first-stage hydrogenation reactor for use as a raw material.

According to a preferred embodiment of the present invention, the process further comprises: cooling the second-stage gas phase obtained by gas-liquid separation of the second-stage hydrogenation product in step (3) followed by a third gas-liquid separation to obtain a third gas phase and a third liquid phase, and using part or all of the third gas phase as a circulating hydrogen to mix with the supplementary hydrogen as the hydrogen raw material for the first-stage hydrogenation reactor, optionally, returning the third liquid phase to the second-stage gas-liquid separator for the second-stage gas-liquid separation. It is preferred that the second-stage gas phase obtained by gas-liquid separation of the second-stage hydrogenation product is cooled via a heat exchanger to a temperature of 30-80°C, so that the heat can be effectively removed and the catalytic efficiency can be improved.

According to a preferred embodiment of the present invention, after the gas-liquid separation of the second-stage hydrogenation reaction product, the second-stage gas phase material can be cooled via a heat exchanger, with the cooling temperature being preferably 30-80°C, and the cooled material is further subjected to gas-liquid separation, the obtained gas phase is recycled to step (1), and the obtained liquid phase is returned to the previous gas-liquid separator.

In a specific embodiment of the present invention, the maleic anhydride hydrogenation process comprises performing a two-stage hydrogenation reaction, wherein
(1) the maleic anhydride solution is divided into two streams, wherein, after the first stream is mixed with a part of the second-stage liquid phase material which is cooled or uncooled, the mixture is fed to the first-stage hydrogenation reactor from the upper liquid-phase feed port of the first-stage hydrogenation reactor to contact with the hydrogen raw material for the hydrogenation reaction, wherein the hydrogen raw material is fed to the first-stage hydrogenation reactor from the top gas-phase feed port of the first-stage hydrogenation reactor;
(2) the first-stage hydrogenation product is subjected to cooling and the first-stage gas-liquid separation successively to obtain the first-stage gas phase and the first-stage liquid phase, wherein all of the first-stage gas is fed to the second-stage hydrogenation reactor from the top gas-phase feed port of the second-stage hydrogenation reactor, and after the first-stage liquid phase is mixed with the second stream of the maleic anhydride solution, the mixture is fed to the second-stage hydrogenation reactor from the upper liquid-phase feed port of the second-stage reactor to react with hydrogen, and to convert all the maleic anhydride into succinic anhydride via the hydrogenation reaction; and
(3) the second-stage hydrogenation product is subjected to the second-stage gas-liquid separation to obtain the second-stage gas phase and the second-stage liquid phase, and a part of the second-stage liquid phase is recycled to step (1) for mixing, and optionally part of all of the second-stage gas phase is used as a circulating hydrogen.

In a preferred maleic anhydride hydrogenation process of the present invention, maleic anhydride is used as a raw material for hydrogenation to produce succinic anhydride. The maleic anhydride solution is divided into at least two streams, wherein the first stream is mixed with a part of the second-stage liquid phase material from the second-stage hydrogenation reaction, and then is fed to the first-stage hydrogenation reactor from the upper part of the first-stage hydrogenation reactor, wherein the second stream is mixed with the first-stage liquid phase material from the first-stage hydrogenation reaction, and then is fed to the second-stage hydrogenation reactor from the upper part of the second-stage hydrogenation reactor. After two stages of hydrogenation reaction, all the maleic anhydride is converted into succinic anhydride. In the present invention, the maleic anhydride solution is divided into at least two streams, which are mixed with different materials respectively and then enter the two hydrogenation reactors, so that the amount of maleic anhydride entering the reactor decreases, the heat released in each reactor is reduced, and the operation is flexible and easy to control. At the same time, the maleic anhydride concentration in the incoming maleic anhydride solution does not need to be too low, which reduces the amount of solvent used so that the energy consumption of subsequent solvent recovery is reduced. The present invention has the characteristics of simple process, low investment, strong applicability, easy control, etc.

The maleic anhydride hydrogenation process of the present invention has a good gas-liquid-solid contact and a high effective utilization rate of the catalyst, and saves investment. The operation conditions for the reaction are mild, the reaction can be carried out at about 40°C, and the temperature rise of the reaction bed is low. Compared with the prior art, the reaction temperature is greatly reduced, which is beneficial to improving the catalyst selectivity and extending the catalyst life.

### Process for producing succinic acid

The present invention provides a process for producing succinic acid, comprising the maleic anhydride hydrogenation process according to the present invention. This process easily removes the reaction heat, has a low molar ratio of hydrogen to maleic anhydride, and obtains the desired quality and yield of succinic acid.

The present invention also provides a process for producing succinic acid, comprising a maleic anhydride separation process and a maleic anhydride hydrogenation process, preferably the maleic anhydride hydrogenation process according to the present invention. In the maleic anhydride separation process, a mixture comprising maleic anhydride is fed to a maleic anhydride separation unit comprising an absorption tower and a rectification tower, and undergoes absorption - rectification to obtain a maleic anhydride solution. Thus, the present invention simplifies and improves the maleic anhydride separation process, reduces energy consumption and investment, and increases the yield of succinic anhydride. The combined operation of the maleic anhydride separation process and the maleic anhydride hydrogenation process allows the absorption solvent for the production of maleic anhydride to be directly used as the raw material for the hydrogenation of maleic anhydride to produce succinic anhydride, which helps to build a whole-process production process.

In a specific embodiment of the present invention, the process for producing succinic acid from butane and/or benzene comprises:
1) subjecting butane and/or benzene and oxygen-containing gas to an oxidation reaction in an oxidation reaction unit to obtain an oxidation reaction product;
2) feeding the oxidation reaction product to a maleic anhydride separation unit comprising an absorption tower and a rectification tower to obtain a maleic anhydride solution via absorption-rectification;
3) feeding the maleic anhydride solution to a maleic anhydride hydrogenation reaction unit for a maleic anhydride hydrogenation reaction, preferably the maleic anhydride hydrogenation reaction according to the present invention, to obtain a hydrogenation product;
4) feeding the unrecycled second-stage liquid phase from the second-stage hydrogenation reaction to a succinic anhydride separation unit to separate succinic anhydride and a solvent; optionally, returning the separated solvent to step (2) for recycling as an absorbent for the absorption tower; optionally, using the separated solvent to dilute the maleic anhydride solution from the maleic anhydride separation unit followed by being sent to the maleic anhydride hydrogenation reaction unit;
5) feeding the succinic anhydride to a succinic anhydride hydrolysis unit for hydrolysis and crystallization to obtain a succinic acid product.

The present invention has no special requirements for the oxygen-containing gas. Commonly-used oxygen-containing gases for oxidation can be used in the present invention, such as air and/or oxygen.

The process of the present invention specifically comprises the following steps:
1) feeding butane/benzene and air to an oxidation reaction unit;
2) feeding the oxidation reaction product to a maleic anhydride separation unit to obtain a maleic anhydride solution via absorption and rectification;
3) feeding the maleic anhydride solution to a maleic anhydride hydrogenation reaction unit;
4) passing the unrecycled second-stage liquid phase of the hydrogenation product through a succinic anhydride separation unit to obtain the products of succinic anhydride and a solvent, and returning the separated solvent to step (2) for recycling;
5) feeding the separated succinic anhydride to a hydrolysis unit for hydrolysis- crystallization to obtain a succinic acid product.

According to the present invention, in step 1), butane/benzene and the oxygen-containing gas such as air are fed to the oxidation reaction unit. The design and operation conditions for the oxidation reaction unit are not specifically limited, and can be determined by a person skilled in the art according to professional knowledge and the prior art, for example, an operating temperature for the oxidation reactor of 400~450°C, and an operating pressure of 0.1~0.3MPag, and removal of the reaction heat by a molten salt cooler and a gas cooler.

According to the present invention, in step 2), the maleic anhydride separation unit mainly comprises an absorption tower, a rectification tower, and other devices, such as a heat exchanger, a pump, a tank, a pipeline, etc., which are determined by a person skilled in the art according to the situation and the prior art. The maleic anhydride separation unit can be operated without vacuum. The oxidation reaction product after heat exchange and cooling is fed to the absorption tower from the bottom of the absorption tower, the solvent is fed to the absorption tower from the top of the tower, the material from the top of the absorption tower is sent outside the boundary area, and the rich solvent obtained at the absorption tower kettle is fed to the rectification tower. The material at the top of the rectification tower is withdrawn and sent outside the boundary area, and the material at the tower kettle is sent to the maleic anhydride hydrogenation reaction unit. The rectification tower can remove water, acetic acid, acrylic acid and other substances by rectification, reduce the generation of maleic acid from maleic anhydride, and increase the yield of succinic anhydride.

According to the present invention, optionally, in the rectification tower in step 2), the material from the top of the tower can be withdrawn and sent outside the boundary area, a mixture of maleic anhydride and the solvent is withdrawn from the side line of the tower and sent to the maleic anhydride hydrogenation reaction unit, and the material at the tower kettle is sent to a solvent purification unit.

According to the present invention, in step 2), the operating pressure for the absorption tower is 0.0-1.0MPag, such as 0.0-0.8MPag, the operating temperature is 40-120°C, such as 60-100°C, and the number of theoretical plates is 5-50, such as 15-35.

According to the present invention, in step 2), the operating pressure for the rectification tower is 0.0-1.0MPag, such as 0.0-0.8MPag, the operating temperature is 40-150°C, such as 60-120°C, and the number of theoretical plates is 5-100, for example 15-80.

According to the present invention, in step 2), it is preferred to replenish a fresh absorbent, which is fed to the top of the absorption tower.

According to the present invention, in step 2), the absorbent is preferably a solvent required for the maleic anhydride hydrogenation reaction, for example, one or a mixed solvent of more than one selected from the group consisting of γ-butyrolactone, dibutyl phthalate, diisobutyl hexahydrophthalate, tetrahydrofuran, aromatic hydrocarbons, ethyl acetate, C4 dibasic acid esters, ethanol, isopropanol, hexane, cyclohexane, propylene oxide, benzene, xylene, chlorobenzene, dichlorobenzene, dioxane, and some ketone and ether solvents, etc., preferably γ-butyrolactone, dioxane, and tetrahydrofuran.

According to the present invention, in step 2), the ratio of the absorbent to the solvent for maleic anhydride is not specifically limited and is determined by a person skilled in the art according to professional knowledge and the prior art. Preferably, the ratio of the absorbent to the oxidation reactant is 0.1-5.

According to the present invention, optionally, in step 2), a part of the material from the absorption tower kettle is cooled to 30-80°C and then returned to the absorption tower, and the rest is sent to the rectification tower.

According to the present invention, optionally, in step 2), the material from the top of the absorption tower is cooled to 20-50°C via a heat exchanger, and then passes through a gas-liquid separator, wherein the gas phase is sent outside the boundary area, and the liquid phase is sent to rectification tower.

According to a preferred embodiment of the present invention, in the maleic anhydride separation unit, the operation conditions for the absorption tower include: a pressure of 0.0-1.0MPag, a temperature of 40-120°C, and a number of theoretical plates of 5-50; and the absorbent being one or a mixed solvent of more than one selected from the group consisting of γ-butyrolactone, dibutyl phthalate, diisobutyl hexahydrophthalate, tetrahydrofuran, aromatic hydrocarbons, ethyl acetate, C4 dibasic acid esters, ethanol, isopropanol, hexane, cyclohexane, propylene oxide, benzene, xylene, chlorobenzene, dichlorobenzene, dioxane, ketones and ethers, preferably γ-butyrolactone, dioxane, and/or tetrahydrofuran.

In step 3) of the process for producing succinic acid in the present invention, the maleic anhydride hydrogenation reaction unit and the maleic anhydride hydrogenation reaction process are not specifically limited, and are determined by a person skilled in the art according to professional knowledge and the prior art. According to a preferred embodiment of the present invention, the maleic anhydride hydrogenation reaction in step 3) is the maleic anhydride hydrogenation process according to the present invention as described above.

According to a specific embodiment of the present invention, the hydrogenation reaction in step 3) in the process for producing succinic acid comprises:
(1) feeding the maleic anhydride solution and the hydrogen raw material to a first-stage hydrogenation reactor from an upper liquid-phase feed port and a top gas-phase feed port of the first-stage hydrogenation reactor respectively for a first-stage hydrogenation reaction to obtain a first-stage hydrogenation product;
(2) feeding the first-stage hydrogenation product to a second-stage hydrogenation reactor for a second-stage hydrogenation reaction to obtain a second-stage hydrogenation product; optionally, before entering the second-stage hydrogenation reactor, subjecting the first-stage hydrogenation product to a first-stage gas-liquid separation to obtain a first-stage gas phase and a first-stage liquid phase, and then feeding the first-stage gas phase and the first-stage liquid phase from a top gas-phase feed port and an upper liquid-phase feed port of the second-stage hydrogenation reactor respectively; and
(3) subjecting the second-stage hydrogenation product to a second-stage gas-liquid separation to obtain a second-stage gas phase and a second-stage liquid phase, and returning a part of the second-stage liquid phase to step (1) to be mixed with the maleic anhydride solution for the first-stage hydrogenation reaction, optionally using part or all of the second-stage gas phase as a circulating hydrogen;
(4) sending the rest of the second-stage liquid phase material to the succinic anhydride separation unit.

According to a preferred embodiment of the present invention, the hydrogenation reaction in step 3) in the process for producing succinic acid comprises:
(1) dividing the maleic anhydride solution into two streams, wherein, after the first stream is mixed with a part of the second-stage liquid phase material which is cooled or uncooled, the mixture is fed to the first-stage hydrogenation reactor from the upper liquid-phase feed port of the first-stage hydrogenation reactor to contact with the hydrogen raw material for the hydrogenation reaction;
(2) subjecting the first-stage hydrogenation product to cooling and the first-stage gas-liquid separation successively to obtain a first-stage gas phase and a first-stage liquid phase, wherein all of the first-stage gas is fed to the second-stage hydrogenation reactor from the top gas-phase feed port of the second-stage hydrogenation reactor, and after the first-stage liquid phase is mixed with the second stream of maleic anhydride solution, the mixture is fed to the second-stage hydrogenation reactor from the upper liquid-phase feed port of the second-stage reactor, to convert all the maleic anhydride into succinic anhydride via the hydrogenation reaction; and
(3) subjecting the second-stage hydrogenation product to the second-stage gas-liquid separation to obtain the second-stage gas phase and the second-stage liquid phase, recycling a part of the second-stage liquid phase to step (1) for mixing, and optionally using part of all of the second-stage gas phase as a circulating hydrogen;
(4) sending the rest of the second-stage liquid phase material to the succinic anhydride separation unit.

According to a preferred embodiment of the present invention, 20-90% by weight of the second-stage liquid phase material from the second-stage hydrogenation reaction is returned to step (1) for use as raw material, and the rest of the second-stage liquid phase is sent to a light removal tower of the succinic anhydride separation unit. The maleic anhydride hydrogenation process of the present invention is used to obtain the succinic anhydride product through the joint operation of a light removal tower and a heavy removal tower. The process is simple, easy to operate and control, and the succinic anhydride product has a high purity.

According to a preferred embodiment of the present invention, preferably, 10-80% by weight of the second-stage liquid phase from the second-stage hydrogenation product after gas-liquid separation is sent to the light removal tower, and the rest of the second-stage liquid phase is first cooled to 40-80°C by a cooler, and then mixed with the maleic anhydride solution to enter the first-stage hydrogenation reactor for recycling.

According to a preferred embodiment of the present invention, in step (2), the operation conditions for the first-stage hydrogenation reactor include: a temperature of 30-100°C, preferably 40-80°C; a reaction pressure of 0.1-10MPa, preferably 0.5-5MPa; and a space velocity of 0.5-8h⁻¹, preferably 0.5-5h⁻¹.

According to a preferred embodiment of the present invention, in step (2), the operation conditions for the second-stage hydrogenation reactor include: a temperature of 30-100°C, preferably 40-80°C; a reaction pressure of 0.1-10MPa, preferably 0.5-5MPa; and a space velocity of 0.5-8h⁻¹, preferably 0.5-5h⁻¹.

According to the present invention, in step 4), the succinic anhydride separation unit mainly comprises a light removal tower and a heavy removal tower. The material from the maleic anhydride hydrogenation reaction unit is fed to the light removal tower, and the material from the tower kettle of the light removal tower is fed to the heavy removal tower, wherein the solvent is withdrawn from the top of the heavy removal tower, the succinic anhydride is withdrawn from the side line of the tower, and a heavy component is withdrawn from the tower kettle.

According to the present invention, optionally, in step 4), the succinic anhydride separation unit further comprises a solvent recovery tower. The material from the maleic anhydride hydrogenation reaction unit is fed to the light removal tower, the material from the tower kettle of the light removal tower is fed to the solvent recovery tower, wherein the solvent is withdrawn from the top of the solvent recovery tower, and the material from the tower kettle of the solvent recovery tower is sent to the heavy removal tower, wherein the succinic anhydride is withdrawn from the top of the heavy removal tower, and a heavy component is withdrawn from the tower kettle of the heavy removal tower. Thus, the yield and purity of succinic anhydride can be improved.

According to a preferred embodiment of the present invention, in step 4), the succinic anhydride separation unit comprises a light removal tower and a heavy removal tower connected in series. The rest of the second-stage liquid phase material from the maleic anhydride hydrogenation reaction unit is fed to the light removal tower, the material from the tower kettle of the light removal tower is fed to the heavy removal tower, wherein the solvent is withdrawn from the top of the heavy removal tower, the succinic anhydride is withdrawn from the side line of the tower, and a heavy component is withdrawn from the tower kettle. Preferably, the succinic anhydride separation unit comprises a light removal tower, a solvent recovery tower and a heavy removal tower connected in series. The rest of the second-stage liquid phase material from the maleic anhydride hydrogenation reaction unit is fed to the light removal tower, the material from the tower kettle of the light removal tower is fed to the solvent recovery tower, a heavy component is withdrawn from the tower kettle of the solvent recovery tower and is fed to the heavy removal tower, and the succinic anhydride is withdrawn from the top of the heavy removal tower.

According to a preferred embodiment of the present invention, a light component is discharged from the top of the light removal tower, and the material from the tower kettle of the light removal tower is fed to the heavy removal tower, wherein a by-product is discharged from the top of the heavy removal tower, a heavy component is discharged from the tower kettle, and the succinic anhydride is withdrawn from the side line.

In the present invention, the light component refers to dissolved hydrogen and a small amount of solvents such as γ-butyrolactone, tetrahydrofuran, etc.

In the present invention, the purpose of the light removal tower is to remove hydrogen and a small amount of solvents such as γ-butyrolactone, tetrahydrofuran, etc. There are no special requirements for its arrangements and operation conditions, as long as the purpose of the present invention can be achieved.

In the present invention, the purpose of the heavy removal tower is to remove the solvent from the top of the tower, remove the heavy component produced by polymerization from the tower kettle, and withdraw the succinic anhydride that meets the requirements from the side line. There are no special requirements for its arrangements and operation conditions, as long as the purpose of the present invention can be achieved.

The present invention has no special requirements for the operation conditions for the light removal tower, and all commonly-used operation conditions for the light removal tower are applicable to the present invention. In the present invention, preferably in step 4), the operating pressure for the light removal tower is 0.5-20KPa, preferably 6-15KPa; the operating temperature is 30-150°C, preferably 80-130°C; and the number of theoretical plates is 10-80, preferably 20-60.

The present invention has no special requirements for the operation conditions for the heavy removal tower, and all commonly-used operation conditions for heavy removal tower are applicable to the present invention. In the present invention, preferably in step 4), the operating pressure for the heavy removal tower is 0.5-20KPa, preferably 3-15KPa; the operating temperature is 30-250°C, such as 30-150°C and preferably 100-130°C, such as 50- 250°C and preferably 100-200°C; and the number of theoretical plates is 10-80, preferably 20-60.

According to the present invention, in step 4), the operating pressure for the solvent recovery tower is 0.5-20KPa, preferably 3-15KPa; the operating temperature is 30-150°C, preferably 100-130°C; and the number of theoretical plates is 10-80, preferably 20 -60.

According to the present invention, in step 4), it is preferred that the separated solvent needs to be heat exchanged to the absorption temperature and recycled to the absorption tower in step 2) for recycling.

According to the present invention, in step 5), the hydrolysis unit is not specifically limited in the present invention and is determined by a person skilled in the art according to professional knowledge and the prior art, for example, hot water is used for hydrolysis, the hydrolysis temperature is 50-95°C, and the hydrolysis pressure is 0.1-0.3MPa.

### Liquid phase hydrogenation reaction system

The present invention provides a liquid phase hydrogenation reaction system, which system comprises:
a first-stage hydrogenation reactor, comprising a top gas-phase feed port, an upper liquid-phase feed port and a bottom discharge port;
a first-stage hydrogenation product cooler and a first-stage gas-liquid separator, which are successively connected in series to the bottom discharge port of the first-stage hydrogenation reactor;
a second-stage hydrogenation reactor, which is connected in series to the first-stage gas-liquid separator, and comprises a too aas-phase feed port, an upper liquid-phase feed port, and a bottom discharge port;
a second-stage gas-liquid separator, which is connected in series to the bottom discharge port of the second-stage hydrogenation reactor; and
a liquid-phase raw material supply pipeline, which is connected to the upper liquid-phase feed port of the first-stage hydrogenation reactor and optionally to the upper liquid-phase feed port of the second-stage hydrogenation reactor.

In an embodiment of the present invention, the liquid-phase raw material supply pipeline is connected to the upper liquid-phase feed port of the first-stage hydrogenation reactor. Preferably, the liquid-phase raw material supply pipeline is connected to both the upper liquid-phase feed port of the first-stage hydrogenation reactor and the upper liquid-phase feed port of the second-stage hydrogenation reactor.

In an embodiment of the present invention, the top gas-phase discharge port of the first-stage gas-liquid separator is connected to the top gas-phase feed port of the second-stage hydrogenation reactor via a pipeline. Thus the top gas phase of the first-stage gas-liquid separator can enter the second-stage hydrogenation reactor from the top gas-phase feed port.

In an embodiment of the present invention, the bottom liquid-phase discharge port of the first-stage gas-liquid separator is connected to the upper liquid-phase feed port of the second-stage hydrogenation reactor via a pipeline. Thus the bottom liquid phase of the first-stage gas-liquid separator can enter the second-stage hydrogenation reactor from the upper liquid-phase feed port.

In an embodiment of the present invention, the top gas-phase discharge port of the second-stage gas-liquid separator is connected to the top gas-phase feed port of the first-stage hydrogenation reactor via a pipeline. Thus the top gas phase of the second-stage gas-liquid separator can return to the first-stage hydrogenation reactor from the top gas-phase feed port.

In an embodiment of the present invention, the bottom liquid-phase discharge port of the second-stage gas-liquid separator is connected to the upper liquid-phase feed port of the first-stage hydrogenation reactor via a pipeline. Thus the bottom liquid phase of the second-stage gas-liquid separator can return to the first-stage hydrogenation reactor from the upper liquid-phase feed port.

In an embodiment of the present invention, it is preferred to arrange a circulating material cooler on the connecting pipeline between the bottom liquid-phase discharge port of the second-stage gas-liquid separator and the upper liquid-phase feed port of the first-stage hydrogenation reactor.

In an embodiment of the present invention, it is preferred to arrange a circulating gas cooler on the connecting pipeline between the top gas-phase discharge port of the second-stage gas-liquid separator and the top gas-phase feed port of the first-stage hydrogenation reactor.

In an embodiment of the present invention, it is preferred to successively arrange a second-stage cooler and a third gas-liquid separator in series at the top gas-phase discharge port of the second-stage gas-liquid separator. The gas-phase discharge port of the third gas-liquid separator is connected to the top gas-phase feed port of the first-stage hydrogenation reactor via a pipeline; and the bottom liquid-phase discharge port of the third gas-liquid separator is connected to the liquid-phase feed port of the second-stage gas-liquid separator. Thus the gas phase of the third gas-liquid separator can return to the first-stage hydrogenation reactor from the top gas-phase feed port, and the bottom liquid phase of the third gas-liquid separator can return to the liquid-phase feed port of the second-stage gas-liquid separator for carrying out gas-liquid separation again. Preferably, it is preferred to arrange a circulating gas cooler on the connecting pipeline between the gas-phase discharge port of the third gas-liquid separator and the top gas-phase feed port of the first-stage hydrogenation reactor.

In an embodiment of the present invention, the system further comprises a distributor for distributing the liquid-phase raw material into two streams as required to supply the first-stage hydrogenation reactor and the second-stage hydrogenation reactor.

In an embodiment of the present invention, optionally, the first-stage hydrogenation reactor and the second-stage hydrogenation reactor are provided with a raw material dispenser at the top, and the gas phase and the liquid phase enter the first-stage hydrogenation reactor and the second-stage hydrogenation reactor, pass through the dispenser, and then contact with the catalyst for reaction.

In the present invention, in addition to the special configuration of the present invention such as the devices listed in the drawings, devices such as pumps, heat exchangers, tanks, compressors, etc. can also be included as needed, and are arranged by a person skilled in the art as required and professional knowledge.

The liquid phase hydrogenation reaction system of the present invention can enhance catalytic efficiency, improve catalytic conversion rate, and can effectively remove reaction heat, and is suitable for various hydrogenation reactions that require heat removal and enhanced catalytic efficiency. The liquid phase hydrogenation reaction system of the present invention is particularly suitable for maleic anhydride hydrogenation reaction, such as the maleic anhydride hydrogenation process according to the present invention.

For example, the raw material of the liquid phase hydrogenation reaction system of the present invention is divided into two streams to enter each stage of reactor respectively, and after the first-stage hydrogenation reactor, a cooler and a gas-liquid separator are arranged, and the obtained gas phase and liquid phase enter the second-stage hydrogenation reactor respectively, which can improve the catalytic efficiency and effectively remove the heat released by the reaction, with flexible operation and easy control.

The present invention provides the use of the liquid phase hydrogenation reaction system according to the present invention in a maleic anhydride hydrogenation process.

The present invention provides the use of the liquid phase hydrogenation reaction system according to the present invention in a succinic acid production system.

### Succinic acid production system

The present invention provides a system for producing succinic acid from butane and/or benzene, comprising the liquid phase hydrogenation reaction system according to the present invention as a maleic anhydride hydrogenation reaction unit.

The present invention also provides a system for producing succinic acid from butane and/or benzene, comprising a maleic anhydride separation unit and a maleic anhydride hydrogenation reaction unit, preferably the liquid phase hydrogenation reaction system according to the present invention as the maleic anhydride hydrogenation reaction unit, wherein the maleic anhydride separation unit comprises an absorption tower and a rectification tower.

In a specific embodiment of the present invention, the system for producing succinic acid from butane and/or benzene comprises: an oxidation reaction unit, a maleic anhydride separation unit comprising an absorption tower and a rectification tower connected in series, a maleic anhydride hydrogenation reaction unit, a succinic anhydride separation unit, and a succinic anhydride hydrolysis unit, connected in series along the material flow direction;
wherein butane and/or benzene undergo an oxidation reaction with an oxygen-containing gas in the oxidation reaction unit, and are fed to the maleic anhydride separation unit for absorption-rectification to obtain a maleic anhydride solution; the maleic anhydride solution is fed to the maleic anhydride hydrogenation reaction unit for hydrogenation reaction to obtain the hydrogenation product; the hydrogenation product is fed to the succinic anhydride separation unit to separate the succinic anhydride and the solvent; the succinic anhydride is fed to the succinic anhydride hydrolysis unit for hydrolysis and crystallization to obtain the succinic acid product.

The present invention has no special requirements for the oxidation reaction unit, the succinic anhydride separation unit, the succinic anhydride hydrolysis unit, etc. A person skilled in the art can make judgment and choice according to the technologies in the art.

The present invention has no special requirements for the maleic anhydride hydrogenation reaction unit, which can be determined by a person skilled in the art according to professional knowledge and the prior art. According to a preferred embodiment of the present invention, the maleic anhydride hydrogenation reaction unit is the liquid phase hydrogenation reaction system according to the present invention as described above.

According to a specific embodiment of the present invention, the maleic anhydride hydrogenation reaction unit comprises:
a first-stage hydrogenation reactor, a first-stage reaction product cooler and a first-stage gas-liquid separator that are connected in series along the material flow direction; a second-stage hydrogenation reactor connected via a top gas-phase feed port to a gas-phase discharge port of the first-stage gas-liquid separator, and connected via an upper liquid-phase feed port to a liquid phase discharge port of the first-stage gas-liquid separator; and a second-stage gas-liquid separator connected in series to the second-stage hydrogenation reactor;
a liquid-phase raw material supply pipeline connected to an upper liquid-phase feed port of the first-stage hydrogenation reactor and optionally to the upper liquid-phase feed port of the second-stage hydrogenation reactor.

In a specific embodiment of the present invention,
the succinic anhydride separation unit comprises: a light removal tower and a heavy removal tower connected in series; wherein
a feed port of the light removal tower is connected to a liquid phase discharge port of the second-stage gas-liquid separator, and the light removal tower is provided with a top discharge port and a tower kettle discharge port; and
a feed port of the heavy removal tower is connected to the tower kettle discharge port of the light removal tower, and the heavy removal tower is provided with a top discharge port, a bottom discharge port and a side line withdrawal port;
   or
the succinic anhydride separation unit comprises: a light removal tower, a solvent recovery tower and a heavy removal tower connected in series; wherein
a feed port of the light removal tower is connected to a liquid phase discharge port of the second-stage gas-liquid separator, and the light removal tower is provided with a top discharge port and a tower kettle discharge port;
a feed port of the solvent recovery tower is connected to the tower kettle discharge port of the light removal tower, and the solvent recovery tower is provided with a top discharge port and a tower kettle discharge port; and
a feed port of the heavy removal tower is connected to the tower kettle discharge port of the solvent recovery tower, and the heavy removal tower comprises a tower kettle discharge port and a top discharge port.

Hereinafter, exemplary embodiments of the present invention are described in more details with reference to drawings.

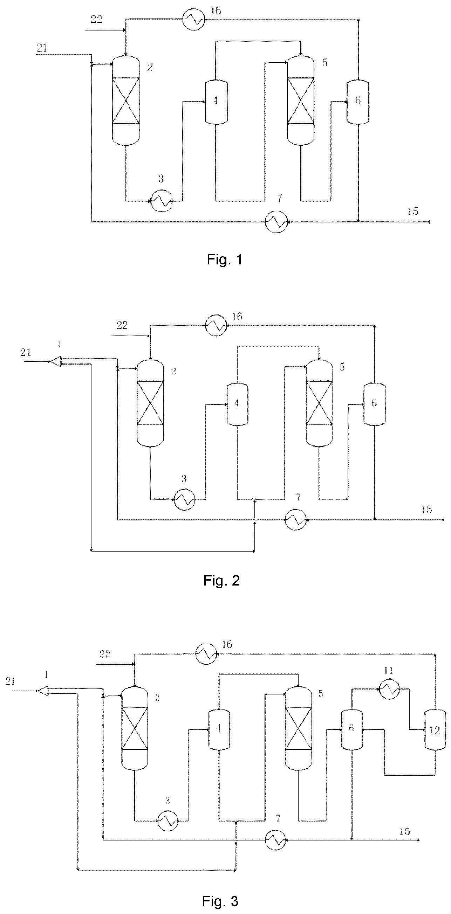

Figures 1-3 exemplarily describe a schematic diagram of the maleic anhydride hydrogenation process according to the present invention and the liquid phase hydrogenation reaction system applicable thereto.

In Figure 1, Figure 2 and Figure 3, 1: a distributor; 2: a first-stage hydrogenation reactor; 3: a first-stage hydrogenation product cooler; 4: a first-stage gas-liquid separator; 5: a second-stage hydrogenation reactor; 6: a second-stage gas-liquid separator; 7: a circulating material cooler; 11: a second-stage cooler; 12: a third gas-liquid separator; 16: a circulating gas cooler; 21: a maleic anhydride solution; 15: a liquid phase product; 22: a supplementary hydrogen.

As shown in Figure 1, in an exemplary embodiment of the present invention, the maleic anhydride hydrogenation process comprises:
(1) after mixing the maleic anhydride solution 21 with a part of the second-stage liquid phase material from the second-stage hydrogenation reaction which is the cooled or uncooled (cooled in the circulating material cooler 7), feeding the mixture to the first-stage hydrogenation reactor 2 from the upper liquid-phase feed port of the first-stage hydrogenation reactor 2, to contact with hydrogen (containing supplementary hydrogen 22 and circulating hydrogen) for hydrogenation reaction, wherein the hydrogen (containing supplementary hydrogen 22 and circulating hydrogen) is fed to the first-stage hydrogenation reactor 2 from the top gas-phase feed port of the first-stage hydrogenation reactor 2;
(2) feeding the first-stage hydrogenation product successively to the first-stage hydrogenation product cooler 3 for cooling and the first-stage gas-liquid separator 4 for a first-stage gas-liquid separation to obtain a first-stage gas phase and a first-stage liquid phase, feeding all the first-stage gas phase to the second-stage hydrogenation reactor 5 from the top gas-phase feed port of the second-stage hydrogenation reactor 5, and feeding the first-stage liquid phase to the second-stage hydrogenation reactor 5 from the upper liquid-phase feed port of the second-stage hydrogenation reactor 5, to react with hydrogen, and to convert all the maleic anhydride into succinic anhydride via the hydrogenation reaction;
(3) feeding the second-stage hydrogenation product to the second-stage gas-liquid separator 6 for a second-stage gas-liquid separation to obtain a second-stage gas phase and the part of the second-stage liquid phase material from the second-stage hydrogenation reaction described in step (1) and a liquid phase product 15, optionally using part or all of the second-stage gas phase as a circulating hydrogen.

As shown in Figures 2-3, in an exemplary embodiment of the present invention, the maleic anhydride hydrogenation process comprises:
(1) dividing the maleic anhydride solution 21 into two streams through a distributor 1, wherein after the first stream is mixed with a part of the second-stage liquid phase material from the second-stage hydrogenation reaction which is cooled or uncooled (cooled in the circulating material cooler 7), feeding the mixture to the first-stage hydrogenation reactor 2 from the upper liquid-phase feed port of the first-stage hydrogenation reactor 2, to contact with hydrogen (containing supplementary hydrogen 22 and circulating hydrogen) for hydrogenation, wherein the hydrogen (containing supplementary hydrogen 22 and circulating hydrogen) is fed to the first-stage hydrogenation reactor 2 from the top gas-phase feed port of the first-stage hydrogenation reactor 2;
(2) feeding the first-stage hydrogenation product successively to the first-stage hydrogenation product cooler 3 for cooling and the first-stage gas-liquid separator 4 for a first-stage gas-liquid separation to obtain a first-stage gas phase and a first-stage liquid phase, feeding all the first-stage gas phase to the second-stage hydrogenation reactor 5 from the top gas-phase feed port of the second-stage hydrogenation reactor 5, mixing the first-stage liquid phase with the second stream of the maleic anhydride solution and then feeding the mixture to the second-stage hydrogenation reactor 6 from the upper liquid-phase feed port of the second-stage hydrogenation reactor 5, to react with hydrogen, and to convert all the maleic anhydride into succinic anhydride via the hydrogenation reaction;
(3) feeding the second-stage hydrogenation product to the second-stage gas-liquid separator 6 for a second-stage gas-liquid separation to obtain a second-stage gas phase and the part of the second-stage liquid phase material from the second-stage hydrogenation reaction described in step (1) and a liquid phase product 15, optionally using part or all of the second-stage gas phase as a circulating hydrogen (as shown in Figure 2); or
feeding the second-stage gas phase obtained by the second-stage gas-liquid separation of the second-stage hydrogenation product to the second-stage cooler 11 for cooling and then to the third gas-liquid separator 12 for a third gas-liquid separation to obtain a third gas phase and a third liquid phase, using part or all of the third gas phase as a circulating hydrogen to mix with supplementary hydrogen 22 and then return to the first-stage hydrogenation reactor 2; optionally, returning the third liquid phase to the second-stage gas-liquid separator 6 for gas-liquid separation (as shown in Figure 3). It is preferred that the second-stage gas phase obtained by the second-stage gas-liquid separation of the second-stage hydrogenation product is cooled to a temperature of 30-80°C in the second-stage cooler 11, so that heat can be effectively removed and the catalytic efficiency can be improved.

As shown in Figures 1-3, in an exemplary embodiment of the present invention, the liquid phase hydrogenation reaction system comprises:
the first-stage hydrogenation reactor 2, the first-stage hydrogenation product cooler 3 and the first-stage gas-liquid separator 4 that are connected in series along the material flow direction;
the second-stage hydrogenation reactor 5 connected via the gas-phase feed port to the gas-phase discharge port of the first-stage gas-liquid separator 4, and connected via the liquid-phase feed port to the liquid phase discharge port of the first-stage gas-liquid separator 4;
and the second-stage gas-liquid separator 6 connected in series to the second-stage hydrogenation reactor 5;
the liquid-phase raw material supply pipeline connected to the liquid-phase feed port of the first-stage hydrogenation reactor 2, or
the liquid-phase raw material supply pipeline connected to both the liquid-phase feed port of the first-stage hydrogenation reactor 2 and the liquid-phase feed port of the second-stage hydrogenation reactor 5.

As shown in Figures 1-3, in the liquid-phase hydrogenation reaction system, the first-stage reaction product cooler 3 and the first-stage gas-liquid separator 4 are successively connected in series to the bottom discharge port of the first-stage hydrogenation reactor 2.

As shown in Figures 1-3, in the liquid phase hydrogenation reaction system, the second-stage gas-liquid separator 6 is connected in series to the bottom discharge port of the second-stage hydrogenation reactor 5.

As shown in Figures 1-3, in the liquid phase hydrogenation reaction system, the first-stage hydrogenation reactor 2 comprises a top gas-phase feed port, an upper liquid-phase feed port, and a bottom discharge port.

As shown in Figures 1-3, in the liquid phase hydrogenation reaction system, the second-stage hydrogenation reactor 5 comprises a top gas-phase feed port, an upper liquid-phase feed port, and a bottom discharge port.

As shown in Figures 1-3, in the liquid phase hydrogenation reaction system, the top gas-phase discharge port of the first-stage gas-liquid separator 4 is connected to the top gas-phase feed port of the second-stage hydrogenation reactor 5 via a pipeline.

As shown in Figures 1-3, in the liquid phase hydrogenation reaction system, the bottom liquid phase discharge port of the first-stage gas-liquid separator 4 is connected to the upper liquid-phase feed port of the second-stage hydrogenation reactor 5 via a pipeline.

As shown in Figures 1-3, in the liquid phase hydrogenation reaction system, the top gas-phase discharge port of the second-stage gas-liquid separator 6 is connected to the top gas-phase feed port of the first-stage hydrogenation reactor 2 via a pipeline.

As shown in Figures 1-3, in the liquid phase hydrogenation reaction system, the bottom liquid phase discharge port of the second-stage gas-liquid separator 6 is connected to the upper liquid-phase feed port of the first-stage hydrogenation reactor 2 via a pipeline.

As shown in Figures 1-3, preferably, a circulating material cooler 7 is provided on the connecting pipeline between the bottom liquid phase discharge port of the second-stage gas-liquid separator 6 and the upper liquid-phase feed port of the first-stage hydrogenation reactor 2.

As shown in Figures 1-3, preferably, a circulating gas cooler 16 is provided on the connecting pipeline between the top gas-phase discharge port of the second-stage gas-liquid separator 6 and the top gas-phase feed port of the first-stage hydrogenation reactor 2.

As shown in Figures 2-3, the liquid phase hydrogenation reaction system further comprises a distributor 1, which is used to distribute the liquid-phase raw material into two streams as required to supply the first-stage hydrogenation reactor 2 and the second-stage hydrogenation reactor 5.

As shown in Figure 3, a second-stage cooler 11 and a third gas-liquid separator 12 are successively arranged in series at the top gas-phase discharge port end of the second-stage gas-liquid separator 6, the gas-phase discharge port of the third gas-liquid separator 12 is connected to the top gas-phase feed port of the first-stage hydrogenation reactor 2 via a pipeline; and the bottom liquid-phase discharge port of the third gas-liquid separator 12 is connected to the liquid-phase feed port of the second-stage gas-liquid separator 6.

As shown in Figure 3, a circulating gas cooler 16 is provided on the connecting pipeline between the gas-phase discharge port of the third gas-liquid separator 12 and the top gas-phase feed port of the first-stage hydrogenation reactor 2.

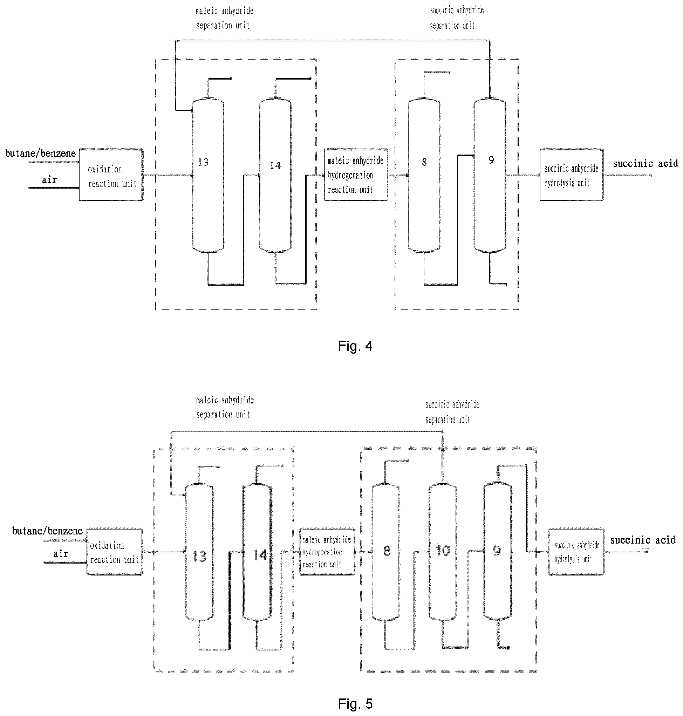

Figures 4-5 exemplarily describe a schematic diagram of a process for producing succinic acid according to the present invention and a succinic acid production system applicable thereto.

In Figures 4 and 5, 8: a light removal tower; 9: a heavy removal tower; 10: a solvent recovery tower; 13: an absorption tower; 14: a rectification tower.

As shown in Figures 4-5, in an exemplary embodiment of the present invention, the process for producing succinic acid from butane and/or benzene comprises:
1) subjecting butane and/or benzene and air to oxidation reaction in an oxidation reaction unit to obtain an oxidation reaction product;
2) feeding the oxidation reaction product to the absorption tower (13), feeding an absorbent to the absorption tower (13) from the top of the absorption tower for absorption, feeding the rich solvent obtained at the absorption tower kettle to the rectification tower (14) for rectification, and sending the tower kettle material of the rectification tower to a maleic anhydride hydrogenation reaction unit;
3) performing hydrogenation reaction in the maleic anhydride hydrogenation reaction unit to obtain a hydrogenation product (as shown in Figures 1-3);
4) feeding the hydrogenation product to the light removal tower (8), withdrawing a light component from the top of the light removal tower (8), and feeding the tower kettle material of the light removal tower (8) to the heavy removal tower (9), withdrawing a solvent from the top of the heavy removal tower (9) to return to the absorption tower (13) for recycling as the absorbent, withdrawing succinic anhydride from the side line of the heavy removal tower (9), and withdrawing a heavy component from the tower kettle of the heavy removal tower (9) (as shown in Figure 4), optionally, using the solvent withdrawn from the heavy removal tower (9) to dilute the maleic anhydride solution from the maleic anhydride separation unit (not shown); or feeding the hydrogenation product to the light removal tower (8), withdrawing a light component from the top of the light removal tower (8), feeding the tower kettle material of the light removal tower (8) to the solvent recovery tower (10), withdrawing a solvent from the top of the solvent recovery tower (10) to return the same to the absorption tower (13) for recycling as the absorbent, and withdrawing a heavy component from the tower kettle of the solvent recovery tower (10) to feed the same to the heavy removal tower (9), withdrawing succinic anhydride from the top of the heavy removal tower (9) (as shown in Figure 5), optionally, using the solvent withdrawn from the solvent recovery tower (10) to dilute the maleic anhydride solution from the maleic anhydride separation unit (not shown);
5) feeding the succinic anhydride to a succinic anhydride hydrolysis unit for hydrolysis and crystallization to obtain the succinic acid product.

As shown in Figure 2, in an exemplary embodiment of the present invention, the maleic anhydride hydrogenation reaction in step 3) of the process for producing succinic acid from butane and/or benzene comprises:
(1) dividing the maleic anhydride solution 21 into two streams through a distributor 1, wherein after the first stream is mixed with a part of the liquid phase material from the second-stage hydrogenation reaction which is cooled or uncooled, feeding the mixture to the first-stage hydrogenation reactor 2 from the upper liquid-phase feed port of the first-stage hydrogenation reactor 2, to contact with hydrogen for hydrogenation, wherein the hydrogen is fed to the first-stage hydrogenation reactor 2 from the top gas-phase feed port of the first-stage hydrogenation reactor 2;
(2) feeding the first-stage hydrogenation product successively to the first-stage hydrogenation product cooler 3 for cooling and the first-stage gas-liquid separator 4 for a first-stage gas-liquid separation, feeding all the first-stage gas phase from the gas-liquid separation to the second-stage hydrogenation reactor 5 from the top gas-phase feed port of the second-stage hydrogenation reactor 5, mixing the first-stage liquid phase from the gas-liquid separation with the second stream and then feeding the mixture to the second-stage hydrogenation reactor 5 from the upper liquid-phase feed port of the second-stage hydrogenation reactor 5, to react with hydrogen, and to convert all the maleic anhydride into succinic anhydride via the hydrogenation reaction;
(3) subjecting the second-stage hydrogenation product to the second-stage gas-liquid separation to obtain the second-stage gas phase and the second-stage liquid phase, returning a part of the liquid phase material from the second-stage hydrogenation reaction to step (3), and optionally using part or all of the gas phase of the second-stage hydrogenation product as a circulating hydrogen;
(4) sending the rest of the liquid phase material from the second-stage hydrogenation reaction to the light removal tower (8), withdrawing a light component from the top of the light removal tower (8), and sending the tower kettle material of the light removal tower (8) to the heavy removal tower (9);
(5) withdrawing succinic anhydride from the side line of the heavy removal tower (9), withdrawing by-products including solvents such as γ-butyrolactone from the top of the tower, and withdrawing a heavy component including polymer from the tower kettle.

As shown in Figures 4-5, in an exemplary embodiment of the present invention, the system for producing succinic acid from butane and/or benzene comprises:
an oxidation reaction unit, a maleic anhydride separation unit comprising an absorption tower (13) and a rectification tower (14) connected in series, a maleic anhydride hydrogenation reaction unit, a succinic anhydride separation unit, and a succinic anhydride hydrolysis unit, connected in series along the material flow direction;
wherein butane and/or benzene undergo an oxidation reaction with oxygen in the oxidation reaction unit, and are fed to the maleic anhydride separation unit for absorption-rectification to obtain a maleic anhydride solution; the maleic anhydride solution is fed to the maleic anhydride hydrogenation reaction unit for hydrogenation reaction to obtain a hydrogenation product; the hydrogenation product is fed to the succinic anhydride separation unit to separate the succinic anhydride and the solvent; the succinic anhydride is fed to the succinic anhydride hydrolysis unit for hydrolysis and crystallization to obtain the succinic acid product.

As shown in Figure 4, the succinic anhydride separation unit comprises: a light removal tower (8) and a heavy removal tower (9) connected in series; wherein the feed port of the light removal tower (8) is connected to the liquid phase discharge port of the second-stage gas-liquid separator, and the light removal tower (8) is provided with a top discharge port and a tower kettle discharge port; the feed port of the heavy removal tower (9) is connected to the tower kettle discharge port of the light removal tower (8), and the heavy removal tower (8) is provided with a top discharge port, a bottom discharge port and a side line withdrawal port.

As shown in Figure 5, the succinic anhydride separation unit comprises: a light removal tower (8), a solvent recovery tower (10) and a heavy removal tower (9) connected in series; wherein the feed port of the light removal tower (8) is connected to the liquid phase discharge port of the second-stage gas-liquid separator, and the light removal tower (8) is provided with a top discharge port and a tower kettle discharge port; the feed port of the solvent recovery tower (10) is connected to the tower kettle discharge port of the light removal tower (8), and the solvent recovery tower (10) is provided with a top discharge port and a tower kettle discharge port; the feed port of the heavy removal tower (9) is connected to the tower kettle discharge port of the solvent recovery tower (10), and the heavy removal tower (9) comprises a tower kettle discharge port and a top discharge port.

### Examples

The following examples use the following catalysts:

### Hydrogenation catalyst S1

With reference to the process of the preparation of a hydrogenation catalyst as described in Preparation Example 1 of the applicant's patent application CN114433100 (A), the hydrogenation catalyst S1 was prepared according to the following procedure:
(1) weighing 50.00g of basic nickel carbonate (nickel content of 45% by weight), 9.16g of Cu(NO₃)₂·3H₂O, 49.91g of ethylenediamine tetraacetic acid, 500g of deionized water and 100g of 25% by weight aqueous ammonia and mixing them, and introducing ammonia gas, to adjust the pH value of the solution to 10.5, stirring at 45°C until all solids were dissolved, thereby obtaining a nickel-copper ammonia complex solution;
(2) weighing 458.31g of a silica sol to mix with the nickel-copper ammonia complex solution obtained in step (1) to obtain a mixed liquid;
(3) aging the mixed liquid at a temperature of 60°C for 14h under stirring, followed by drying at 120°C for 12h to obtain a catalyst precursor;
(4) saturating the catalyst precursor with a cerium nitrate solution containing 11.41g of Ce(NO₃)₃·6H₂O to obtain a matrix catalyst;
(5) drying the matrix catalyst at 115°C for 12 hours, followed by calcining at 400°C for 4h, to shape and obtain the catalyst S1.

Based on the total weight of the catalyst S1, the catalyst S1 comprised: 19 wt% of NiO, 2 wt% of CuO, 3 wt% of CeOz and 76 wt% of SiOz.

### Hydrogenation catalyst S2

With reference to the process of the preparation of a hydrogenation catalyst as described in Example 1 of the applicant's patent application CN114433127 (A), the hydrogenation catalyst S2 was prepared according to the following procedure:
(1) weighing 10.90g of Ni(NO₃)3·6H₂O and 5.04g of Ce(NO₃)₃·6H₂O, and dissolving them in water to a volume of 50.0ml, then immersing 50g of the carrier SiOz (having a specific surface area of 300m²/g, and a water absorption rate of 1.0mL/g) in the nickel nitrate-cerium nitrate mixed solution, stirring homogeneously, and allowing to stand for aging for 4h, followed by oven drying at 120°C for 12 hours, and finally calcining in air at 450°C for 4h to obtain a composite oxide carrier E;
(2) adding the composite oxide carrier E to 100 ml of a ruthenium metal solution having a Ru content of 0.02g/L; under stirring condition, adding dropwise an aqueous ammonia having a mass concentration of 25% to adjust the pH value of the solution and maintain it at 9; after reacting at 55°C for 6h, performing filtration, followed by oven drying at 110°C for 12h, and finally calcining at 500°C in air for 4 h to obtain the finished catalyst S2.

In the catalyst S2, based on the mass of the catalyst carrier SiO₂, the mass fraction of Ni in the catalyst was 7% of the carrier mass, the mass fraction of CeOz was 4% of the carrier mass, and the mass fraction of Ru was 0.4% of the carrier mass.

### Example 1

According to the invention, maleic anhydride hydrogenation reaction was carried out. γ-butyrolactone was used as a solvent, and the content of maleic anhydride in the maleic anhydride solution was 10% by weight. The maleic anhydride solution was mixed with a part of the second-stage liquid phase material recycled from the second-stage hydrogenation reaction, and then fed to the first-stage hydrogenation reactor from the upper liquid-phase feed port of the reactor. The molar ratio of the total hydrogen amount of the circulating hydrogen and the supplementary fresh hydrogen to the total maleic anhydride in the incoming maleic anhydride solution was 12.

In the first-stage hydrogenation reactor, the space velocity of the first-stage hydrogenation reactor was 2.5h⁻¹, the reaction temperature was 40°C, and the reaction pressure was 1.5MPa. The first-stage hydrogenation reaction product was cooled to 40°C. After the first-stage gas-liquid separation, all the first-stage gas phase and all the first-stage liquid phase were fed to the second-stage hydrogenation reactor from the top gas-phase feed port and the upper liquid-phase feed port of the reactor respectively. In the second-stage hydrogenation reactor, the space velocity of the second-stage hydrogenation reactor was 1h⁻¹, the reaction temperature was 45°C, and the reaction pressure was 1.3MPa. After the second-stage hydrogenation reaction product underwent the second-stage gas-liquid separation, 1 vol.% of the second-stage gas phase was withdrawn and vented, and the rest of the gas phase was sent to the first-stage hydrogenation reactor together with the supplementary fresh hydrogen, and 65% by weight of the liquid phase in the second-stage liquid phase after the gas-liquid separation was sent to the subsequent separation system (such as a light removal tower), and 35% of the liquid phase was heat exchanged to 40°C, mixed with the maleic anhydride solution, and then fed to the first-stage hydrogenation reactor.

The catalysts loaded in the first-stage and second-stage hydrogenation reactors were both the hydrogenation catalyst S1 (a catalyst of Ni active component).

After the two-stage hydrogenation reaction, the total conversion rate of maleic anhydride was 99.91%, and the total selectivity of succinic anhydride was 99.85%.

### Example 2

According to the invention, maleic anhydride hydrogenation reaction was carried out. γ-butyrolactone was used as a solvent, and the content of maleic anhydride in the maleic anhydride solution was 10% by weight. The maleic anhydride solution was divided into two streams according to the proportions of 50% by weight and 50% by weight, wherein the first stream (50% by weight of the maleic anhydride solution) was mixed with a part of the second-stage liquid phase material recycled from the second-stage hydrogenation reaction, and then fed to the first-stage hydrogenation reactor from the upper liquid-phase feed port of the reactor. The second stream (50% by weight of the maleic anhydride solution) was mixed with the first-stage liquid phase product from the first-stage hydrogenation reaction, and then fed to the second-stage hydrogenation reactor from the upper liquid-phase feed port of the second-stage hydrogenation reactor. The molar ratio of the total hydrogen amount of the circulating hydrogen and the supplementary fresh hydrogen to the total maleic anhydride in the incoming maleic anhydride solution was 10.

In the first-stage hydrogenation reactor, the space velocity of the first-stage hydrogenation reactor was 2.5h⁻¹, the reaction temperature was 40°C, and the reaction pressure was 1.5MPa. The first-stage hydrogenation reaction product was cooled to 40°C. After the first-stage gas-liquid separation, all the first-stage gas phase were fed to the second-stage hydrogenation reactor from the top gas-phase feed port of the second-stage hydrogenation reactor, and the first-stage liquid phase was mixed with the second stream of maleic anhydride solution and then fed to the second-stage hydrogenation reactor from the upper liquid-phase feed port of the second-stage hydrogenation reactor. In the second-stage hydrogenation reactor, the space velocity of the second-stage hydrogenation reactor was 1h⁻¹, the reaction temperature was 42°C, and the reaction pressure was 1.3MPa. After the second-stage hydrogenation reaction product passed through the second-stage gas-liquid separator, the second-stage gas phase was cooled to 40°C and then sent to the first-stage hydrogenation reactor together with the supplementary fresh hydrogen, 65% by weight of the liquid phase in the second-stage liquid phase after the gas-liquid separation was sent to the subsequent separation system (such as a light removal tower), and 35% by weight of the liquid phase was heat exchanged to 40°C, mixed with the first stream of maleic anhydride solution, and then fed to the first-stage hydrogenation reactor.

The catalysts loaded in the first-stage and second-stage hydrogenation reactors were both the hydrogenation catalyst S1 (a catalyst of Ni active component).

After the two-stage hydrogenation reaction, the total conversion rate of maleic anhydride was 99.91%, and the total selectivity of succinic anhydride was 99.83%.

### Example 3

According to the invention, the maleic anhydride hydrogenation reaction was carried out. Dioxane was used as a solvent, and the content of maleic anhydride in the maleic anhydride solution was 18% by weight. The maleic anhydride solution was divided into two streams according to the proportions of 20% by weight and 80% by weight, wherein the first stream (20% by weight of the maleic anhydride solution) was mixed with a part of the second-stage liquid phase material recycled from the second-stage hydrogenation reaction, and then fed to the first-stage hydrogenation reactor from the upper liquid-phase feed port of the first-stage hydrogenation reactor, and the second stream (80% by weight of the maleic anhydride solution) was mixed with the first-stage liquid phase product from the first-stage hydrogenation reaction, and then fed to the second-stage hydrogenation reactor from the upper liquid-phase feed port of the second-stage hydrogenation reactor. The molar ratio of the total hydrogen amount of the circulating hydrogen and the supplementary fresh hydrogen to the total maleic anhydride in the incoming maleic anhydride solution was 30.

In the first-stage hydrogenation reactor, the space velocity of the first-stage hydrogenation reactor was 1.8h⁻¹, the reaction temperature was 40°C, and the reaction pressure was 1.3MPa. The first-stage hydrogenation reaction product was cooled to 45°C. After the first-stage gas-liquid separation, all the first-stage gas phase was fed to the second-stage hydrogenation reactor from the top gas-phase feed port of the second-stage hydrogenation reactor, and the first-stage liquid phase was mixed with the second stream of maleic anhydride solution and then fed to the second-stage hydrogenation reactor from the upper liquid-phase feed port of the second-stage hydrogenation reactor. In the second-stage hydrogenation reactor, the space velocity of the second-stage hydrogenation reactor was 1.2h⁻¹, the reaction temperature was 48°C, and the reaction pressure was 1.2MPa. After the second-stage hydrogenation reaction product passed through the second-stage gas-liquid separator, the second-stage gas phase was sent to the first-stage hydrogenation reactor together with the supplementary fresh hydrogen, 60% by weight of the liquid phase in the second-stage liquid phase after the gas-liquid separation was sent to the subsequent separation system (such as a light removal tower), and 40% by weight of the liquid phase was heat exchanged to 40°C, mixed with the first stream of maleic anhydride solution, and then fed to the first-stage hydrogenation reactor.

The catalysts loaded in the first-stage and second-stage hydrogenation reactors were both the hydrogenation catalyst S1 (a catalyst of Ni active component).

After the two-stage hydrogenation reaction, the total conversion rate of maleic anhydride was 99.83%, and the total selectivity of succinic anhydride was 99.85%.

### Example 4

According to the invention, the maleic anhydride hydrogenation reaction was carried out. Hexane was used as a solvent, and the content of maleic anhydride in the maleic anhydride solution was 25% by weight. The maleic anhydride solution was divided into two streams according to the proportions of 40% by weight and 60% by weight, wherein the first stream (40% by weight of the maleic anhydride solution) was mixed with a part of the second-stage liquid phase material recycled from the second-stage hydrogenation reaction, and then fed to the first-stage hydrogenation reactor from the upper liquid-phase feed port of the first-stage reactor, and the second stream (60% by weight of the maleic anhydride solution) was mixed with the first-stage liquid phase product from the first-stage hydrogenation reaction, and then fed to the second-stage hydrogenation reactor from the upper liquid-phase feed port of the second-stage hydrogenation reactor. The molar ratio of the total hydrogen amount of the circulating hydrogen and the supplementary fresh hydrogen to the total maleic anhydride in the incoming maleic anhydride solution was 40.

In the first-stage hydrogenation reactor, the space velocity of the first-stage hydrogenation reactor was 3h⁻¹, the reaction temperature was 40°C, and the reaction pressure was 1.7MPa. The first-stage hydrogenation reaction product was cooled to 42°C. After the first-stage gas-liquid separation, all the first-stage gas phase was fed to the second-stage hydrogenation reactor from the top gas-phase feed port of the second-stage hydrogenation reactor, and the first-stage liquid phase was mixed with the second stream of maleic anhydride solution and then fed to the second-stage hydrogenation reactor from the upper liquid-phase feed port of the second-stage hydrogenation reactor. In the second-stage hydrogenation reactor, the space velocity of the second-stage hydrogenation reactor was 0.8h⁻¹, the reaction temperature was 45°C, and the reaction pressure was 1.5MPa. After the second-stage hydrogenation reaction product passed through the second-stage gas-liquid separator, the gas phase was further cooled to 40°C and passed through the third gas-liquid separator, wherein the obtained gas phase was sent to the first-stage hydrogenation reactor together with the supplementary fresh hydrogen; 50% by weight of the liquid phase in the second-stage liquid phase from the second-stage gas-liquid separator was sent to the subsequent separation system (such as a light removal tower); and 50% by weight of the liquid phase was heat exchanged to 40°C, mixed with the first stream of maleic anhydride solution, and then fed to the first-stage hydrogenation reactor.

The catalysts loaded in the first-stage and second-stage hydrogenation reactors were both the hydrogenation catalyst S2 (a catalyst of Ni active component).

After the two-stage hydrogenation reaction, the total conversion rate of maleic anhydride was 99.89%, and the total selectivity of succinic anhydride was 99.78%.

### Comparative example 1

The maleic anhydride hydrogenation reaction of Example 1 was used, wherein γ-butyrolactone was used as a solvent and the maleic anhydride content in the maleic anhydride solution was 10% by weight, with the difference in that all the liquid phase material from the second-stage hydrogenation reaction product was sent to the subsequent separation unit, and was not recycled back to mix with the maleic anhydride solution.

In the first-stage hydrogenation reactor, the space velocity of the first-stage hydrogenation reactor was 2.5h⁻¹, the reaction temperature was 40°C, and the reaction pressure was 1.5MPa. The first-stage hydrogenation reaction product was cooled to 40°C. After the first-stage gas-liquid separation, all the first-stage gas phase and all the first-stage liquid phase were fed to the second-stage hydrogenation reactor from the top gas-phase feed port and the upper liquid-phase feed port of the reactor respectively. In the second-stage hydrogenation reactor, the space velocity of the second-stage hydrogenation reactor was 1h⁻¹, the reaction temperature was 45°C, and the reaction pressure was 1.3MPa. After the second-stage hydrogenation reaction product underwent the second-stage gas-liquid separation, 1 vol.% of the second-stage gas phase was withdrawn and vented, and the rest of the gas phase was sent to the first-stage hydrogenation reactor together with the supplementary fresh hydrogen, all the second-stage liquid phase after the gas-liquid separation was sent to the subsequent separation system (such as a light removal tower).

The catalysts loaded in the first-stage and second-stage hydrogenation reactors were both the hydrogenation catalyst S1 (a catalyst of Ni active component).

After the two-stage hydrogenation reaction, the total conversion rate of maleic anhydride was 99.91%, and the total selectivity of succinic anhydride was 99.85%.

In Comparative example 1, in order to ensure that the outlet temperature of the hydrogenation reactor was the same as that of the example, the molar ratio of the total hydrogen amount of the circulating hydrogen and the supplementary fresh hydrogen to the total maleic anhydride in the incoming maleic anhydride solution needed to be increased by at least 78%, and the hydrogen/anhydride ratio increased greatly. The energy consumption increased significantly, and only considering the energy consumption of the circulating hydrogen compressor, the energy consumption of this compressor increased by 84.8%.

### Comparative example 2

The maleic anhydride hydrogenation reaction of Example 2 was used, wherein γ-butyrolactone was used as a solvent, the maleic anhydride content in the maleic anhydride solution was 10% by weight, and the maleic anhydride solution was divided into two streams according to the proportions of 50% by weight and 50% by weight, with the difference in that all the liquid phase material from the second-stage hydrogenation reaction product was sent to the subsequent separation unit (such as a light removal tower) and was not recycled back to mix with the first stream of maleic anhydride solution.

After the two-stage hydrogenation reaction, the total conversion rate of maleic anhydride was 99.91%, and the total selectivity of succinic anhydride was 99.83%.

In Comparative example 2, in order to ensure that the outlet temperature of the hydrogenation reactor was the same as that of the example, the molar ratio of the total hydrogen amount of the circulating hydrogen and the supplementary fresh hydrogen to the total maleic anhydride in the incoming maleic anhydride solution needed to be increased by at least 79%, and the hydrogen/anhydride ratio increased greatly. The energy consumption increased significantly, and only considering the energy consumption of the circulating hydrogen compressor, the energy consumption of this compressor increased by 84.0%.

### Comparative example 3

The maleic anhydride hydrogenation reaction of Example 3 was used, wherein Dioxane was used as a solvent, the maleic anhydride content in the maleic anhydride solution was 18% by weight, and the maleic anhydride solution was divided into two streams according to the proportions of 20% by weight and 80% by weight, with the difference in that all the liquid phase material from the second-stage hydrogenation reaction product was sent to the subsequent separation unit (such as a light removal tower) and was not recycled back to mix with the first stream of maleic anhydride solution.

After the two-stage hydrogenation reaction, the total conversion rate of maleic anhydride was 99.83%, and the total selectivity of succinic anhydride was 99.85%.

In Comparative example 3, in order to ensure that the outlet temperature of the hydrogenation reactor was the same as that of the example, the molar ratio of the total hydrogen amount of the circulating hydrogen and the supplementary fresh hydrogen to the total maleic anhydride in the incoming maleic anhydride solution needed to be increased by at least 120%, and the hydrogen/anhydride ratio increased greatly. The energy consumption increased significantly, and only considering the energy consumption of the circulating hydrogen compressor, the energy consumption of this compressor increased by 168.4%.

### Comparative example 4

The maleic anhydride hydrogenation reaction of Example 4 was used, wherein Hexane was used as a solvent, the maleic anhydride content in the maleic anhydride solution was 25% by weight, and the maleic anhydride solution was divided into two streams according to the proportions of 40% by weight and 60% by weight, with the difference in that all the liquid phase material from the second-stage hydrogenation reaction product was sent to the subsequent separation unit (such as a light removal tower) and was not recycled back to mix with the first stream of maleic anhydride solution.

After the two-stage hydrogenation reaction, the total conversion rate of maleic anhydride was 99.83%, and the total selectivity of succinic anhydride was 99.85%.

In Comparative example 4, in order to ensure that the outlet temperature of the hydrogenation reactor was the same as that of the example, the molar ratio of the total hydrogen amount of the circulating hydrogen and the supplementary fresh hydrogen to the total maleic anhydride in the incoming maleic anhydride solution needed to be increased by at least 125%, and the hydrogen/anhydride ratio increased greatly. The energy consumption increased significantly, and only considering the energy consumption of the circulating hydrogen compressor, the energy consumption of this compressor increased by 171.7%.

In Comparative examples 1-4, when the liquid phase material from the second-stage hydrogenation reaction product was not recycled but all entered the subsequent separation unit, if the same low hydrogen/anhydride ratios as those in Examples 1-4 were used respectively, the heat released in hydrogenation reactor could not be effectively withdrawn, causing the reactor outlet temperature to be much higher than that in the examples. It can be seen from the comparison of the above examples with the comparative examples that, by partial recycling of the liquid phase material from the second-stage hydrogenation reaction product, the present invention could effectively remove the reaction heat, reduce the molar hydrogen/anhydride ratio of the total hydrogen amount of the circulating hydrogen and the supplementary fresh hydrogen to the total maleic anhydride in the incoming maleic anhydride solution, reduce energy consumption and achieve the desired maleic anhydride conversion rate and succinic anhydride selectivity.

### Example 5

According to the present invention, succinic acid was produced from butane as a raw material.

Butane and air were mixed and fed to the oxidation reaction unit. The oxidation reaction temperature was 420°C and the reaction pressure was 0.2MPag. The oxidation reaction product was fed to the absorption tower of the maleic anhydride separation unit and entered from the tower kettle of the absorption tower, and γ-butyrolactone was used as a solvent and entered the absorption tower from the top of the absorption tower. The absorption tower had a total of 18 theoretical plates, with an operating temperature of 75°C and an operating pressure of 0.06 MPag. A tail gas was withdrawn from the top of the absorption tower, and the rich solvent at the tower kettle was fed to the rectification tower. The rectification tower had a total of 20 theoretical plates, an operating temperature of 95°C, and an operating pressure of 0.003 MPag. A light component was withdrawn from the top of the rectification tower, and the material at the tower kettle was a maleic anhydride solution having a maleic anhydride content of 35% by weight, which was diluted to obtain a maleic anhydride solution having a maleic anhydride content of 10% by weight that is then sent to the maleic anhydride hydrogenation reaction unit.

The maleic anhydride hydrogenation reaction unit adopted two stages of hydrogenation reactors, wherein the maleic anhydride hydrogenation reaction was performed according to the process of Example 1. After the two stages of hydrogenation reaction, the total conversion rate of maleic anhydride was 99.91%, and the total selectivity of succinic anhydride was 99.85%.

The liquid phase material from the maleic anhydride hydrogenation reaction unit was fed to the succinic anhydride separation unit. It first passed through the light removal tower to separate a light component from the top of the tower, and the material from the tower kettle was sent to the heavy removal tower. γ-butyrolactone was withdrawn from the top of the heavy removal tower, a part of the γ-butyrolactone was returned to the absorption tower of the maleic anhydride separation unit for recyclcling, and the rest of γ-butyrolactone was used as a solvent to dilute the maleic anhydride solution having a maleic anhydride content of 35% by weight from the maleic anhydride separation unit to obtain the maleic anhydride solution having a maleic anhydride content of 10% via mixing, which then entered the hydrogenation reactor. A heavy component was withdrawn from the tower kettle of the heavy removal tower, and succinic anhydride was withdrawn from the side line and sent to the succinic anhydride hydrolysis unit.

The light removal tower had 26 theoretical plates, the tower top pressure of 10KPa, and the operating temperature of 100°C. The heavy removal tower had 25 theoretical plates, the tower top pressure of 3KPa, and the operating temperature of 105°C. The obtained succinic anhydride had a purity of 99.9%.

The hydrolysis kettle of the succinic anhydride hydrolysis unit has the operating pressure of 0.12MPa, and the operating temperature of 80°C. After centrifugal separation and drying, succinic acid product was obtained. The succinic acid product had a purity of 99.9%.

### Example 6

According to the present invention, succinic acid was produced from butane as a raw material.

The oxidation reaction-maleic anhydride separation was carried out according to the process of Example 5 to obtain a maleic anhydride solution having a maleic anhydride content of 10% by weight, and γ-butyrolactone was used as the solvent.

The maleic anhydride hydrogenation reaction unit adopted two stages of hydrogenation reactors, wherein the maleic anhydride hydrogenation reaction was performed according to the process of Example 2. After the two stages of hydrogenation reaction, the total conversion rate of maleic anhydride was 99.91 %, and the total selectivity of succinic anhydride was 99.83%.

After separation and hydrolysis, a succinic acid product having a product purity of 99.9% was obtained.

### Example 7

According to the present invention, succinic acid was produced from butane as a raw material.

Dioxane was used as an absorbent, and the oxidation reaction-maleic anhydride separation was carried out according to the process of Example 5 to obtain a maleic anhydride solution having a maleic anhydride content of 18% by weight.

The maleic anhydride hydrogenation reaction unit adopted two stages of hydrogenation reactors, wherein the maleic anhydride hydrogenation reaction was performed according to the process of Example 3. After the two stages of hydrogenation reaction, the total conversion rate of maleic anhydride was 99.83%, and the total selectivity of succinic anhydride was 99.85%.

After separation and hydrolysis, a succinic acid product having a product purity of 99.9% was obtained.

### Example 8

According to the present invention, succinic acid was produced from butane as a raw material.

Hexane was used as an absorbent, the oxidation reaction-maleic anhydride separation was carried out according to the process of Example 5 to obtain a maleic anhydride solution having a maleic anhydride content of 25% by weight, and the solvent was hexane.

The maleic anhydride hydrogenation reaction unit adopted two stages of hydrogenation reactors, wherein the maleic anhydride hydrogenation reaction was performed according to the process of Example 4. After the two stages of hydrogenation reaction, the total conversion rate of maleic anhydride was 99.89%, and the total selectivity of succinic anhydride was 99.78%.

After separation and hydrolysis, a succinic acid product having a product purity of 99.9% was obtained.

### Comparative example 5

According to the prior art, succinic acid was produced from butane as a raw material.

Butane and air were mixed and fed to the oxidation reaction unit. The oxidation reaction temperature was 420°C and the reaction pressure was 0.2MPag. The oxidation reaction product was fed to the absorption tower of the maleic anhydride separation unit and fed from the tower kettle of the absorption tower, and dibutyl phthalate was used as an absorbent and fed to the absorption tower from the top of the absorption tower. The absorption tower had a total of 20 theoretical plates, an operating temperature of 90°C and an operating pressure of 0.06MPag. A tail gas was withdrawn from the top of the absorption tower and sent outside the boundary area, and the rich solvent at the tower kettle was fed to a stripping tower. The stripping tower had a total of 25 theoretical plates, an operating temperature of 142°C and an operating pressure of 12KPa. The material from the top of the stripping tower was sent to the light component tower, and the material at the stripping tower kettle was sent to the absorption tower for recycling. The light component tower had a total of 20 theoretical plates, an operating temperature of 40°C and an operating pressure of 10KPa. The material from the top of the light component tower was sent to the outside of the boundary area, and the material at the tower kettle was sent to the product refining tower. The product refining tower had a total of 25 plates, an operating temperature of 132°C and an operating pressure of 10KPa. The maleic anhydride product was withdrawn from the upper side line of the product refining tower and sent to the maleic anhydride hvdroaenation reaction unit and the material at the tower kettle was heat exchanged to 50°C and returned to the absorption tower for recycling.

The operation conditions of the maleic anhydride hydrogenation reaction unit were the same as those in Example 5, except that γ-butyrolactone/dioxane needed to be introduced from the outside as a solvent. After the two stages of hydrogenation reaction, the total conversion rate of maleic anhydride was 99.50%, and the total selectivity of succinic anhydride was 99%.

The succinic anhydride separation unit and hydrolysis unit were the same as those in Example 5. The uccinic acid product had a purity of 99.5%. In the maleic anhydride separation unit, as compared to Comparative Example 5, Example 5 had two less tower devices, and saved at least 4 heat exchangers, 8 pumps and other auxiliary devices. The maleic anhydride separation unit of Example 5 was operated at a pressure above normal pressure, while in Comparative Example 5, except the absorption tower, all other tower devices were operated under reduced pressure. Therefore, Example 5 could save equipment investment compared with Comparative Example 5. In addition, by analyzing the maleic anhydride separation unit from the perspective of energy consumption, compared with Comparative Example 5, the energy consumption of Example 5 could be reduced by approximately 35%, which was equivalent to saving approximately 68kg standard oil/ton C4.

It can be seen that in the process of the prior art, the operation of the maleic anhydride separation unit is complex, and additional absorbent and solvent need to be introduced, which increases process cost and energy consumption. In contrast, the maleic anhydride separation unit of the present invention is simple in process flow, saves equipment investment and is conducive to saving energy consumption.

Moreover, the succinic acid production process according to the present invention has established a whole-process production process, achieved the combined use of the maleic anhydride separation process and the maleic anhydride hydrogenation process, and obtained a high-purity succinic acid product. The preferred embodiments of the present invention are described in detail above, but the present invention is not limited thereto. Within the scope of the technical concept of the present invention, many simple modifications can be made to the technical solution of the present invention, including the combination of various technical features in any other suitable manner, and these simple modifications and combinations should also be regarded as the contents disclosed by the present invention, and belong to the protection scope of the present invention.

## Claims

1. A process for producing succinic anhydride by hydrogenation of maleic anhydride, comprising the following steps:
(1) feeding a maleic anhydride solution and a hydrogen raw material to a first-stage hydrogenation reactor from an upper liquid-phase feed port and a top gas-phase feed port of the first-stage hydrogenation reactor respectively for a first-stage hydrogenation reaction to obtain a first-stage hydrogenation product;
(2) feeding the first-stage hydrogenation product to a second-stage hydrogenation reactor for a second-stage hydrogenation reaction to obtain a second-stage hydrogenation product; optionally, before entering the second-stage hydrogenation reactor, subjecting the first-stage hydrogenation product to a first-stage gas-liquid separation to obtain a first-stage gas phase and a first-stage liquid phase, and then feeding the first-stage gas phase and the first-stage liquid phase from a top gas-phase feed port and an upper liquid-phase feed port of the second-stage hydrogenation reactor respectively; and
(3) subjecting the second-stage hydrogenation product to a second-stage gas-liquid separation to obtain a second-stage gas phase and a second-stage liquid phase, and returning a part of the second-stage liquid phase to step (1) to be mixed with the maleic anhydride solution for the first-stage hydrogenation reaction, optionally using part or all of the second-stage gas phase as a circulating hydrogen.

2. The process according to claim 1, wherein in step (1),
the maleic anhydride solution is a mixture of maleic anhydride and a solvent, wherein the solvent is one or more of acetic anhydride, gamma-butyrolactone, dioxane, tetrahydrofuran, aromatic hydrocarbons, ethyl acetate, C4 dibasic acid esters, ethanol, isopropanol, hexane, cyclohexane, propylene oxide, ketones and ethers; and/or
the maleic anhydride solution has a maleic anhydride concentration of 1-90% by weight, preferably 5-40% by weight; and/or
the molar ratio of the total amount of hydrogen to the total maleic anhydride in the maleic anhydride solution is 5-100, preferably 10-40; and/or
the operation conditions for the first-stage hydrogenation reactor include: a temperature of 30-100°C, preferably 40-80°C; and/or a reaction pressure of 0.1-10MPa, preferably 0.5-5MPa; and/or a space velocity of 0.5-8h⁻¹; and/or
the hydrogen raw material is a mixed hydrogen gas of the circulating hydrogen in step (3) and a supplementary hydrogen.

3. The process according to any one of claims 1-2, wherein in step (1), the maleic anhydride solution is divided into at least two streams, wherein the first stream is mixed with the part of the second-stage liquid phase in step (3) for the first-stage hydrogenation reaction, and the second stream is used for the second-stage hydrogenation reaction;
preferably, the first stream and the second stream are each 5-95% by weight relative to the maleic anhydride solution; more preferably, the first stream is 20-60% by weight, and the second stream is 40-80% by weight.

4. The process according to any one of claims 1-3, wherein in step (2),
operation conditions for the second-stage hydrogenation reactor include: a temperature of 30-100°C, preferably 40-80°C; and/or a pressure of 0.1-10MPa, preferably 0.5-5MPa; and/or a space velocity of 0.5-5h⁻¹; and/or
the first-stage hydrogenation product is cooled before the first-stage gas-liquid separation.

5. The process according to any one of claims 1-4, wherein in step (3),
said part of the second-stage liquid phase is cooled before being mixed, preferably, cooled to 30-80°C, preferably cooled to 40-60°C; and/or
20-90% by weight of the second-stage liquid phase is returned to step (1) for use as a raw material, preferably 30-70% by weight of the second-stage liquid phase is returned to step (1) for use as a raw material, and the rest is sent to a subsequent separation system as a liquid phase product; and/or
0.5-2% by weight of the second-stage gas phase is withdrawn as fuel gas, and the rest is used as a circulating hydrogen.

6. The process according to any one of claims 1-5, wherein the process further comprises:
cooling the second-stage gas phase in step (3) followed by a third gas-liquid separation to obtain a third gas phase and a third liquid phase, and using part or all of the third gas phase as a circulating hydrogen to mix with the supplementary hydrogen as the hydrogen raw material for the first-stage hydrogenation reactor;
optionally, returning the third liquid phase to the second-stage gas-liquid separator for the second-stage gas-liquid separation;
preferably, cooling the second-stage gas phase to a temperature of 30-80°C.

7. The process according to any one of claims 1-6, wherein
(1) the maleic anhydride solution is divided into two streams, wherein, after the first stream is mixed with the part of the second-stage liquid phase which is cooled or uncooled, the mixture is fed to the first-stage hydrogenation reactor from the upper liquid-phase feed port of the first-stage hydrogenation reactor to contact with the hydrogen raw material for the hydrogenation reaction, wherein the hydrogen raw material is fed to the first-stage hydrogenation reactor from the top gas-phase feed port of the first-stage hydrogenation reactor;
(2) the first-stage hydrogenation product is subjected to cooling and the first-stage gas-liquid separation successively to obtain the first-stage gas phase and the first-stage liquid phase, wherein all of the first-stage gas is fed to the second-stage hydrogenation reactor from the top gas-phase feed port of the second-stage hydrogenation reactor, and after the first-stage liquid phase is mixed with the second stream, the mixture is fed to the second-stage hydrogenation reactor from the upper liquid-phase feed port of the second-stage reactor to react with hydrogen;
(3) the second-stage hydrogenation product is subjected to the second-stage gas-liquid separation to obtain the second-stage gas phase and the second-stage liquid phase, and a part of the second-stage liquid phase is recycled to step (1) for mixing, and optionally part of all of the second-stage gas phase is used as a circulating hydrogen.

8. A process for producing succinic acid, **characterized in that** the process comprises the process for producing succinic anhydride by hydrogenation of maleic anhydride according to any one of claims 1-7.

9. The process according to claim 8, **characterized in that** the process comprises:
1) subjecting butane and/or benzene and oxygen-containing gas to an oxidation reaction in an oxidation reaction unit to obtain an oxidation reaction product;
2) feeding the oxidation reaction product to a maleic anhydride separation unit comprising an absorption tower and a rectification tower to obtain a maleic anhydride solution via absorption-rectification;
3) feeding the maleic anhydride solution to a maleic anhydride hydrogenation reaction unit for the hydrogenation reaction as defined in any one of claims 1-7;
4) feeding the unrecycled second-stage liquid phase from the second-stage hydrogenation reaction to a succinic anhydride separation unit to separate succinic anhydride and a solvent; optionally, returning the separated solvent to step (2) for recycling as an absorbent;
5) feeding the succinic anhydride to a succinic anhydride hydrolysis unit for hydrolysis and crystallization to obtain a succinic acid product.

10. The process according to claim 9, wherein in the maleic anhydride separation unit:
operation conditions for the absorption tower include: a pressure of 0.0-1.0MPag, a temperature of 40-120°C, and a number of theoretical plates of 5-50;
the absorbent is one or a mixed solvent of more than one selected from the group consisting of γ-butyrolactone, dibutyl phthalate, diisobutyl hexahydrophthalate, tetrahydrofuran, aromatic hydrocarbons, ethyl acetate, C4 dibasic acid esters, ethanol, isopropanol, hexane, cyclohexane, propylene oxide, benzene, xylene, chlorobenzene, dichlorobenzene, dioxane, ketones and ethers, preferably γ-butyrolactone, dioxane, and/or tetrahydrofuran;
operation conditions for the rectification tower include: a pressure of 0.0-1.0MPag, a temperature of 40-150°C, and a number of theoretical plates of 5-100.

11. The process according to any one of claims 9-10, wherein in step 2),
a part of the material from the absorption tower kettle is cooled to 30-80°C and then returned to the absorption tower, and the rest is sent to the rectification tower; and/or
the material from the top of the absorption tower is cooled to 20-50°C via a heat exchanger, and
then passes through a gas-liquid separator, wherein the gas phase is sent outside the boundary area, and the liquid phase is sent to the rectification tower.

12. The process according to any one of claims 9-11, wherein in step 4),
the succinic anhydride separation unit comprises a light removal tower and a heavy removal tower connected in series, wherein the rest of the second-stage liquid phase from the second-stage hydrogenation reaction is fed to the light removal tower, and the material from the tower kettle of the light removal tower is fed to the heavy removal tower, wherein the solvent is withdrawn from the top of the heavy removal tower, the succinic anhydride is withdrawn from the side line of the tower, and a heavy component is withdrawn from the tower kettle;
preferably, the succinic anhydride separation unit comprises a light removal tower, a solvent recovery tower and a heavy removal tower connected in series, wherein the rest of the second-stage liquid phase from the second-stage hydrogenation reaction is fed to the light removal tower, the material from the tower kettle of the light removal tower is fed to the solvent recovery tower, a heavy component is withdrawn from the tower kettle of the solvent recovery tower and is fed to the heavy removal tower, and the succinic anhydride is withdrawn from the top of the heavy removal tower.

13. The process according to claim 12, wherein
operation conditions for the light removal tower include: a pressure of 0.5-20KPa, a temperature of 30-150°C, and a number of theoretical plates of 10-80; and/or
operation conditions for the heavy removal tower include: a pressure of 0.5-20KPa, a temperature of 30-250°C, and a number of theoretical plates of 10-80; and/or
operation conditions for the solvent recovery tower include: a pressure of 0.5-20KPa, a temperature of 30-150°C, and a number of theoretical plates of 10-80.

14. A liquid phase hydrogenation reaction system, applicable to the process according to any one of claims 1-7, **characterized in that** the system comprises:
a first-stage hydrogenation reactor, comprising a top gas-phase feed port, an upper liquid-phase feed port and a bottom discharge port;
a first-stage hydrogenation product cooler and a first-stage gas-liquid separator, which are successively connected in series to the bottom discharge port of the first-stage hydrogenation reactor;
a second-stage hydrogenation reactor, which is connected in series with the first-stage gas-liquid separator, and comprises a top gas-phase feed port, an upper liquid-phase feed port, and a bottom discharge port;
a second-stage gas-liquid separator, which is connected in series with the bottom discharge port of the second-stage hydrogenation reactor; and
a liquid-phase raw material supply pipeline, which is connected to the upper liquid-phase feed port of the first-stage hydrogenation reactor and optionally to the upper liquid-phase feed port of the second-stage hydrogenation reactor.

15. The hydrogenation reaction system according to claim 14, wherein
a top gas-phase discharge port of the first-stage gas-liquid separator is connected to the top gas-phase feed port of the second-stage hydrogenation reactor via a pipeline; and/or
a bottom liquid-phase discharge port of the first-stage gas-liquid separator is connected to the upper liquid-phase feed port of the second-stage hydrogenation reactor via a pipeline; and/or
a top gas-phase discharge port of the second-stage gas-liquid separator is connected to the top gas-phase feed port of the first-stage hydrogenation reactor via a pipeline; and/or
a bottom liquid-phase discharge port of the second-stage gas-liquid separator is connected to the upper liquid-phase feed port of the first-stage hydrogenation reactor via a pipeline;
preferably, a circulating material cooler is arranged on the connecting pipeline between the bottom liquid-phase discharge port of the second-stage gas-liquid separator and the upper liquid-phase feed port of the first-stage hydrogenation reactor;
preferably, a circulating gas cooler is arranged on the connecting pipeline between the top gas-phase discharge port of the second-stage gas-liquid separator and the top gas-phase feed port of the first-stage hydrogenation reactor.

16. The hydrogenation reaction system according to claim 14 or 15, the system further comprising: a distributor for distributing the liquid-phase raw material into two streams as required to supply the first-stage hydrogenation reactor and the second-stage hydrogenation reactor; and/or
a second-stage cooler and a third gas-liquid separator successively arranged in series at the top gas-phase discharge port of the second-stage gas-liquid separator, wherein a gas-phase discharge port of the third gas-liquid separator is connected to the top gas-phase feed port of the first-stage hydrogenation reactor via a pipeline; and a bottom liquid-phase discharge port of the third gas-liquid separator is connected to the liquid-phase feed port of the second-stage gas-liquid separator;
preferably, a circulating gas cooler is arranged on the connecting pipeline between the gas-phase discharge port of the third gas-liquid separator and the top gas-phase feed port of the first-stage hydrogenation reactor.

17. A system for producing succinic acid, applicable to the process according to any one of claims 8-13, and **characterized in that** the system comprises the liquid phase hydrogenation reaction system according to any one of claims 14-16 as a maleic anhydride hydrogenation reaction unit.

18. The system according to claim 17, **characterized in that** the system comprises:
an oxidation reaction unit, a maleic anhydride separation unit comprising an absorption tower and a rectification tower connected in series, the maleic anhydride hydrogenation reaction unit,
a succinic anhydride separation unit, and a succinic anhydride hydrolysis unit, connected in series along the material flow direction;
wherein butane and/or benzene undergo an oxidation reaction with an oxygen-containing gas in the oxidation reaction unit, and are fed to the maleic anhydride separation unit for absorption-rectification to obtain a maleic anhydride solution; the maleic anhydride solution is fed to the maleic anhydride hydrogenation reaction unit for hydrogenation reaction to obtain the hydrogenation product; the hydrogenation product is fed to the succinic anhydride separation unit to separate the succinic anhydride and the solvent; the succinic anhydride is fed to the succinic anhydride hydrolysis unit for hydrolysis and crystallization to obtain the succinic acid product.

19. The system according to claim 18, wherein
the succinic anhydride separation unit comprises: a light removal tower and a heavy removal tower connected in series; wherein
a feed port of the light removal tower is connected to the liquid phase discharge port of the second-stage gas-liquid separator, and the light removal tower is provided with a top discharge port and a tower kettle discharge port; and
a feed port of the heavy removal tower is connected to the tower kettle discharge port of the light removal tower, and the heavy removal tower is provided with a top discharge port, a bottom discharge port and a side line withdrawal port;
or
the succinic anhydride separation unit comprises: a light removal tower, a solvent recovery tower and a heavy removal tower connected in series; wherein
a feed port of the light removal tower is connected to the liquid phase discharge port of the second-stage gas-liquid separator, and the light removal tower is provided with a top discharge port and a tower kettle discharge port;
a feed port of the solvent recovery tower is connected to the tower kettle discharge port of the light removal tower, and the solvent recovery tower is provided with a top discharge port and a tower kettle discharge port; and
a feed port of the heavy removal tower is connected to the tower kettle discharge port of the solvent recovery tower, and the heavy removal tower comprises a tower kettle discharge port and a top discharge port.
